# EUROPÄISCHE PATENTSCHRIFT

(11) **EP 1 858 428 B1**
(45) Veröffentlichungstag und Bekanntmachung des Hinweises auf die Patenterteilung: **30.11.2016**
(21) Anmeldenummer: 06722983.1
(22) Anmeldetag: 25.01.2006
(51) Int. Cl.: A61B 18/12, H03B 1/02, A61B 18/14, H03L 5/00, A61B 17/00, A61B 5/05, A61B 18/00

(54) **HF-CHIRURGIEEINRICHTUNG**
HIGH-FREQUENCY SURGICAL DEVICE
DISPOSITIF CHIRURGICAL HAUTE FREQUENCE

(30) Priorität: 26.01.2005 DE 102005003707; 06.06.2005 DE 102005025946
(43) Veröffentlichungstag der Anmeldung: 28.11.2007
(73) Patentinhaber: ERBE Elektromedizin GmbH, 72072 Tübingen (DE)
(72) Erfinder: EISELE, Florian, 72074 Tübingen (DE)
(74) Vertreter: Bohnenberger, Johannes
(86) Internationale Anmeldenummer: PCT/EP2006/000645
(87) Internationale Veröffentlichungsnummer: WO 2006/079524

(56) Entgegenhaltungen:
- WO-A-02/11634
- WO-A-93/03677
- DE-A1- 10 102 254
- DE-A1- 19 542 419
- DE-A1- 19 643 127
- US-A1- 2002 165 530

## Beschreibung

Die Erfindung betrifft ein HF-Chirurgiesystem nach dem Oberbegriff des Patentanspruches 1.

Die Hochfrequenzchirurgie wird seit vielen Jahren sowohl in der Human- als auch in der Veterinärmedizin eingesetzt, um biologisches Gewebe zu koagulieren und/oder zu schneiden. Dabei wird mit Hilfe geeigneter elektrochirurgischer Instrumente hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Eine darauf folgende Erhöhung der Stromdichte bewirkt ein explosionsartiges Verdampfen der Gewebeflüssigkeit und ein Aufreißen der Zellmembranen, wobei das Gewebe vollständig durchtrennt wird.

Für die thermische Behandlung biologischen Gewebes werden sowohl bipolare, als auch monopolare Techniken angewendet. Bei monopolaren Anordnungen wird der von dem HF-Generator an das elektrochirurgische Instrument zugeführte HF-Strom in das zu behandelnde Gewebe über eine differente Elektrode appliziert, wobei der Strompfad durch den Körper eines Patienten bis zu einer indifferenten Neutralelektrode führt und von dort zurück zum HF-Generator. An der differenten Elektrode ist eine hohe Stromdichte pro Flächeneinheit zur Behandlung vorgesehen, während bei der indifferenten Elektrode die Stromdichte pro Flächeneinheit gegenüber der differenten Elektrode deutlich verringert ist. Dies lässt sich durch ein entsprechend großflächiges Auslegen der Neutralelektrode erreichen. Nur so ist gewährleistet, dass an dem Gewebe beim Übergang des Stromes von dem Gewebe auf die Neutralelektrode keine Verletzungen, wie z. B. Verbrennungen auftreten.

Immer mehr an Bedeutung gewinnen auch bipolare Instrumente, die mit zwei voneinander elektrisch isolierten Elektrodenteilen ausgebildet sind. Der Stromweg zwischen den Elektrodenteilen ist damit kalkulierbar und verläuft nicht weite Strecken durch den Körper des Patienten. Damit reduziert sich die Beeinflussung von beispielsweise Herzschrittmachern oder sonstigen Geräten, die während der Operation an dem Patienten angeschlossen sind.

Die monopolare Technik bietet sich insbesondere für die interstitielle Koagulation an, wenn ein sich gleichförmig (z. B. radialsymmetrisch) über das zu behandelnde Gewebe, d. h. über das Zielgewebe ausbreitender HF-Strom für die Behandlung erforderlich ist. So können z. B. Tumoren oder Metastasen behandelt werden, indem das für die monopolare Koagulation geeignete elektrochirurgische Instrument in das zu behandelnden Gewebe, also beispielsweise in einen Tumor, eingebracht (eingestochen) und durch die Applikation des hochfrequenten Stromes, also durch die Koagulation, die Zerstörung des Tumors initiiert wird (Tumordevitalisierung).

Zur Durchführung einer Koagulation und/oder eines Schneidvorganges werden HF-Chirurgieeinrichtungen verwendet, die ein HF-Chirurgiegerät mit einem HF-Generator zur Erzeugung einer hochfrequenten Spannung und damit des hochfrequenten Wechselstromes aufweisen, sowie Schalteinrichtungen und/oder Steuer- und Regeleinrichtung zur Aktivierung bzw. Deaktivierung des HF-Generators.

Die DE 19 542 419 A1 beschreibt einen Hochfrequenzgenerator für die Hochfrequenzchirurgie. Der Hochfrequenzstrom wird über eine Schneidelektrode in das Gewebe des Patienten eingespeist und fließt zu einer Neutralen Elektrode ab. Der Hochfrequenzgenerator verfügt über eine Messeinrichtung, mit deren Hilfe die zur Regelung des Hochfrequenzgenerators erforderlichen Ausgangsgrößen des Hochfrequenzgenerators gemessen werden.

Vorrichtungen der hier angesprochenen Art sind auch aus der US 2002/0165530 A1, der WO 02/11634 A1 und der DE 101 02 254 A1 bekannt.

Bei der monopolaren Koagulation, insbesondere bei der interstitiellen Koagulation, aber auch bei Schneidvorgängen, ist es mittels der vorbekannten HF-Chirurgieeinrichtungen bisher nicht möglich, die Größe einer Koagulationszone vorherzubestimmen bzw. einzuschätzen, da eine Koagulation in dieser Hinsicht nicht steuerbar ist. Vielmehr muss die Größe der zu erwartenden Koagulationszone aufgrund von Erfahrungswerten abgeschätzt und/oder durch bildgebende Verfahren überwacht werden.

Ein ausschließliches Arbeiten mit Erfahrungswerten bedingt jedoch, dass ein Sicherheitszusclilag bei der in das Gewebe einzubringenden Energiemenge berücksichtigt werden muss. Nur mit einem Überschuss an Energie - verbunden mit einer dadurch hervorgerufenen hohen Belastung eines das Zielgewebe umgebenden Gewebes - lässt sich das Risiko einer unvollständigen Koagulation und damit einer unvollständigen Devitalisierung des Zielgewebes vermeiden. Auch die Anwendung bildgebender Verfahren stellt keine zufriedenstellende Lösung dar. Zum einen sind die bildgebenden Verfahren äußerst aufwändig und kostenintensiv, zum anderen lassen sie sich nicht grundsätzlich mit einer HF-Strom-Applikation einsetzen.

Der Erfindung liegt daher die Aufgabe zu Grunde, eine HF-Chirurgieeinrichtung der eingangs genannten Art dahin gehend weiterzubilden, dass Koagulationsvorgänge und/oder Schneidvorgänge optimiert werden und auf einfachste Weise überwachbar sind.

Diese Aufgabe wird durch ein HF-Chirurgiesystem nach Patentanspruch 1 gelöst.

Die beanspruchte Erfindung lässt sich durch die in dieser Schrift, d.h. in der vorliegenden Beschreibung sowie in den Zeichnungen, beschriebenen und dargestellten Ausführungsbeispiele besser verstehen. Im allgemeinen spiegelt die vorliegende Offenbarung bevorzugte Ausführungsbeispiele der Erfindung wieder. Dem aufmerksamen Leser wird jedoch auffallen, dass einige Aspekte der beschriebenen Ausführungsbeispiele über den Schutzumfang der Ansprüche hinausragen. Sofern die beschriebenen Ausführungsbeispiele tatsächlich über den Schutzumfang der Ansprüche hinausragen, sind die beschriebenen Ausführungsbeispiele als zusätzliches Hintergrundinformation zu betrachten und stellen keine Definition der Erfindung per se dar.

Insbesondere wird die Aufgabe durch eine HF-Chirurgieeinrichtung zum Behandeln biologischen Gewebes mittels eines HF-Stromes gelöst, wobei die HF-Chirurgieeinrichtung ein elektrochirurgisclies Instrument mit einer Koagulationselektrode umfasst, sowie ein HF-Chirurgiegerät mit einem HF-Generator zum Erzeugen einer HF-Spannung und zum Zuführen des HF-Stromes zu der Koagulationselektrode des elektrochirurgischen Instruments und mit mindestens einer Steuerungseinrichtung zum Beenden des Koagulationsvorganges. Der Steuerungseinrichtung ist eine Messvorrichtung zugeordnet, welche derart ausgebildet ist, dass sie mindestens einen Messgewebeenergieeintrag in einen definierten Messgewebebereich beschreibenden Messwert erfasst, wobei sie mindestens einen Messwert unmittelbar im Messgewebebereich mittels einer Messelektrode erfasst, wobei die Koagulationselektrode und die Messelektrode derart zueinander angeordnet sind, dass sie den Messgewebebereich hinsichtlich seiner Ausdehnung zumindest eindimensional in einer Richtung abgreifen. Ferner ist der Steuerungseinrichtung eine Recheneinrichtung zugeordnet, welche derart ausgebildet ist, dass sie den Messgewebeenergieeintrag in den definierten Messgewebebereich feststellt und dass sie einen für den Koagulationsvorgang vorgegebenen Endwert für einen Zielgewebeenergieeintrag in einen Zielgewebebereich ermittelt und/oder als gespeicherten Endwert übernimmt. Die Steuerungseinrichtung ist derart ausgebildet, dass sie den HF-Generator auf Basis des Endwertes derart steuert oder regelt, dass der erforderliche HF-Strom dem Zielgewebebereich zugeführt wird und dass sie dann, wenn der Zielgewebeenergieeintrag den Endwert erreicht, ein Abschaltsignal erzeugt.

Im Folgenden wird der Gegenstand der Erfindung insbesondere anhand eines monopolaren Koagulationsvorganges beschrieben. Es sei jedoch angemerkt, dass sich der Gegenstand grundsätzlich auch bei Schneidvorgängen, insbesondere mit der damit verbundenen Koagulation anwenden lässt. Die Erfindung bezieht sich ebenfalls auf bipolare Techniken.

Ein wesentlicher Punkt dieser Offenbarung ist es, den für ein optimales Koagulations- und/oder Schneidergebnis erforderlichen HF-Strom in dem zu behandelnden Gewebe, dem Zielgewebebereich, zur Verfügung zu stellen, indem mindestens ein für den Messgewebeenergieeintrag in den Messgewebebereich charakteristischer Messwert gemessen und daraus ein Energieeintrag festgestellt wird, um darauf und/oder auf Erfahrungswerte basierend den HF-Generator derart zu steuern oder zu regeln, dass der für das optimale Koagulations- und/oder Schneidergebnis erforderliche Energieeintrag durch ein Angleichen des erfolgten Energieeintrags an den vorgegebenen, ggf. ermittelten Endwert für den Zielgewebeenergieeintrag in den Zielgewebebereich erreicht wird.

Die Unterscheidung zwischen Messgewebebereich und Zielgewebebereich, als dem letztendlich zu behandelnden Gewebe, verdeutlicht, dass prinzipiell in einem Bereich gemessen werden kann, der nicht dem zu behandelnden Gewebebereich entspricht. Dies vereinfacht die Messung, und die den Messwert aufnehmenden Bestandteile der Messvorrichtung können unabhängig von Ausmaßen des Zielgewebebereichs an dem zu behandelnden Gewebe angesetzt werden. Da sich eine 'Vermessung' des Zielgewebebereichs als eher schwierig gestaltet, wird in der Regel eine Messung in dem Messgewebebereich durchgeführt. Insofern wird für nachfolgende Beschreibung eine Differenzierung beider Bereiche vorgenommen. Es sei jedoch darauf hingewiesen, dass grundsätzlich auch dann von einem Messgewebebereich gesprochen werden kann, wenn dieser dem Zielgewebebereich entspricht, denn dann ist der Zielgewebebereich der Messgewebebereich. In jedem Fall aber erfolgt die Messung im Gewebe, also nicht (unter Verfälschung durch Leitungsimpedanzen etc.) am Generator selbst.

Die Bestimmung des Energieeintrags in den Messgewebebereich (ggf. auch in den Zielgewebebereich) ist erforderlich, weil nicht vermeidbare Energieverluste, beispielsweise thermische Verluste über den Messgewebebereich bzw. den Zielgewebebereich umgebende Gewebebereiche (letztendlich bis zu einer indifferenten Elektrode hin) oder auch eine elektrische Verlustleistung, verursacht z. B. durch vagabundierende Ströme, kaum erfassbar sind. Ein von einer über die Koagulationselektrode eingebrachter Gesamtenergieeintrag steht nämlich aufgrund dieser Verluste nicht vollständig für den Zielgewebebereich zur Verfügung. Die hier aufgezeigte Messvorrichtung stellt nun darauf ab, mindestens den Messgewebebereich zu 'vermessen', so dass letztendlich beurteilt werden kann, wie viel Energieeintrag für den Zielgewebebereich z. B. erhalten bleiben wird.

Es wird also ein Energieeintrag in den Messgewebebereich ermittelt, um daraus auf den erforderlichen Energieeintrag in den Zielgewebebereich zu schließen. Der erforderliche Energieeintrag in den Zielgewebebereich kann z. B. ermittelt werden, indem von dem bereits erfolgten Energieeintrag in den Messgewebebereich auf den erforderlichen Energieeintrag in den Zielgewebebereich 'hochgerechnet' wird (unter Berücksichtung der oben beschriebenen Verluste). Der Messwert dient also als Grundlage zur Bestimmung des erforderlichen Energieeintrags, wobei z. B. aufgrund des gemessenen Wertes darauf geschlossen werden kann, welche Energiemenge bereits im Zielgewebebereich gewirkt haben muss und welche Energiemenge durch umliegende Gewebebereiche verloren gegangen ist. Der Verlust muss bei der Ermittlung des erforderlichen Energieeintrags in den Zielgewebebereich ausgeglichen werden, ohne das Gewebe zusätzlich zu belasten. Der erforderliche Energieeintrag für den Zielgewebebereich kann aber auch 'von außen' vorgegeben werden, und auf Basis des gemessenen Wertes bzw. des erfolgten Energieeintrages in den Messgewebebereich kann dann der noch notwendige Energieeintrag für eine optimale Behandlung des Zielgewebebereichs aufgrund Steuerung oder Regelung des HF-Generators eingebracht werden. Vorzugsweise erfolgt die Ermittlung des erforderlichen Energieeintrags in den Zielgewebebereich basierend auf Erfahrungswerten, wobei die oben beschriebenen Verluste mit berücksichtigt werden.

Der Endwert für den Zielgewebeenergieeintrag ist im Prinzip vorgegeben, da er beispielsweise auf Erfahrungswerten beruht und/oder aus aktuellen Gewebeparametern ermittelt wird. Der Messgewebeenergieeintrag liefert also einen Istwert, der durch eine entsprechende Steuerung bzw. Regelung an den Endwert, also an einen Sollwert, anzugleichen ist. Der Istwert des Messgewebeenergieeintrags steht mit dem gemessenen Wert in funktionellem Zusammenhang. Genauer heißt dies, dass sich aus einem Istwert der gemessenen Größe der Istwert für den Messgewebeenergieeintrag bestimmen lässt. Der Istwert für den Messgewebeenergieeintrag ist dann an den erforderlichen Endwert für den Zielgewebeenergieeintrag anzugleichen. Dabei wird der HF-Generator und damit der HF-Strom derart gesteuert oder geregelt, dass der Istwert (also der Energieeintrag in den Messgewebebereich) dem ggf. sich ändernden Endwert folgt und zwar so lange, bis der für den optimalen Koagulationsvorgang erforderliche Endwert für den Zielgewebeenergieeintrag erreicht ist. Sobald der Endwert erreicht ist, erzeugt die Steuerungseinrichtung ein Abschaltsignal, das ein Beenden des Koagulationsvorganges bewirkt oder ermöglicht. Es ist natürlich auch möglich, den Istwert anzuzeigen und die Steuerung / Abschaltung des Generators manuell vorzunehmen. Grundsätzlich ist es also auch möglich, die Steuerung / Abschaltung aufgrund Ermittlung des Messwertes und/oder den daraus ermittelbaren, bereits erfolgten Energieeintrag durchzuführen.

Grundsätzlich ließe sich der Endwert für den Zielgewebeenergieeintrag aus dem im Messgewebebereich gemessenen Wert bzw. aus den gemessenen Werten, z. B. durch Extrapolation, ermitteln. Das heißt, aus dem gemessenen Wert oder aus den gemessenen Werten soll hochgerechnet werden, welcher Energieeintrag für den Zielgewebebereich erforderlich ist. Dies ist insbesondere dann leicht möglich, wenn der Messgewebebereich dem Zielgewebebereich entspricht. Zur Präzisierung einer Stromregelung bietet es sich in der Praxis jedoch an, die Ermittlung des Endwertes zumindest zusätzlich unter Bezugnahme auf Erfahrungswerte durchzuführen, die z. B. die oben beschriebenen Verluste mit einbeziehen. Die Erfahrungswerte liegen beispielsweise aus Experimenten oder aus früheren Eingriffen vor und berücksichtigen Gewebeparameter von bereits vermessenen, dem zu behandelnden Gewebe möglichst ähnlichen Gewebearten. Damit lassen sich Endwerte als Sollwerte vorgeben, an die der Energieeintrag in den Messgewebebereich als momentan vorliegender Istwert über die Stromregelung (-Steuerung) anzugleichen ist, um letztendlich den erforderlichen Energieeintrag in den Zielgewebebereich zu erhalten. Letzteres ist insbesondere dann empfehlenswert, wenn sich der Messgewebebereich vom Zielgewebebereich unterscheidet.

Zur Bestimmung des Energieeintrags müssen u. a. der Strom durch den Messgewebebereich und die über dem Messgewebebereich abfallende Spannung bekannt sein. Der Strom durch den Messgewebebereich ist gleich dem Generatorstrom (Leckströme sind bei den zur Koagulation üblicherweise verwendeten Spannungen vernachlässigbar) und kann mit herkömmlicher Technik im Chirurgiegerät erfasst werden. Die Spannung über dem Messgewebebereich hingegen ist nicht ohne Weiteres verfügbar, denn sie unterscheidet sich aufgrund der oben beschriebenen Verluste von der Ausgangsspannung des Chirurgiegerätes um einen Spannungsabfall den der HF-Strom insbesondere in den 'anderen Strukturen' außerhalb des Messgewebebereichs bzw. Zielgewebebereichs zusätzlich verursacht. Dieser Spannungsabfall wird also z. B. durch einen Patientenwiderstand bewirkt, aber auch durch einen Übergangswiderstand, der zwischen der Koagulationselektrode und dem Zielgewebebereich (auch Messgewebebereich) auftritt. Der Begriff 'Patientenwiderstand' meint hier die 'anderen Strukturen', also das nicht zu behandelnde Gewebe, das zwischen dem definierten Zielgewebebereich und der Neutralelektrode als Strompfad zur Verfügung steht, sowie ggf. einen weiteren Übergangswiderstand zwischen diesem Gewebe und der am Patienten angebrachten Neutralelektrode.

Wird über die Messung von Spannung und Strom auf die in das Zielgewebe eingebrachte Leistung abgestellt, so kann es vorteilhaft sein, auch die Zeit, die zur Applikation der Leistung nötig ist, zu ermitteln, da dieser 'Energiezufluss' zusammen mit den oben beschriebenen Verlusten durch das umliegende Gewebe (='Energieabfluss') über die Ausbildung des Temperaturgradienten entscheidet. Der Temperaturgradient wiederum ist entscheidend für die letztendlich interessierende Frage bis zu welcher Stelle im Gewebe eine für die Devitalisierung ausreichende Temperatur erreicht wird.

Im Falle der thermischen Devitalisierung durch den HF-Strom entspricht die Energiemenge der über die Zeit integrierten im Mess- bzw. Zielgewebebereich umgesetzten elektrischen Leistung.

In einer ersten bevorzugten Ausführungsform ist die Recheneinrichtung derart ausgebildet, dass der Endwert für den Zielgewebeenergieeintrag in zeitlich definierten Abständen wiederholt neu ermittelt wird, so dass dieser entsprechend einem bestimmten zeitlichen Ablauf einer Zeitplanregelung vorgegeben wird. Die Zeitplanregelung ist z. B. dann erforderlich, wenn der zu behandelnde Gewebebereich während des Koagulationsvorganges beobachtet und dessen Gewebeveränderungen berücksichtigt werden sollen bzw. wenn aus der Erfahrung bereits bekannt ist, dass der Sollwert einer ständigen Anpassung während der Koagulation bedarf. Die sich während der Koagulation verändernden Gewebeparameter bilden somit die Basis für den weiteren Koagulationsverlauf. Das heißt, der optimale Endwert für den Zielgewebeenergieeintrag kann sich im Laufe der Koagulation derart verändern, dass eine wiederholte Neubestimmung des Endwertes, dem der Istwert folgen soll, erforderlich ist.

Die Zeitplanregelung kann alternativ derart vorgesehen sein, dass eine feste Zeitplanregelung vorab, beispielsweise auf Erfahrungswerte beruhend, vorgegeben wird, wobei der Koagulationsverlauf in dem zu behandelnden Gewebe z. B. unberücksichtigt bleibt. Das heißt, die Recheneinrichtung ist derart ausgebildet, dass der Endwert für den Zielgewebeenergieeintrag aus einem vorgegebenen Sollwertverlauf eingelesen wird. Auch bietet sich eine Kombination aktueller Gewebeparameter mit vorgegebenen Erfahrungswerten an, um einen optimalen Energieeintrag in das zu behandelnde Gewebe zu erzielen.

Eine Zeitplanregelung ermöglicht auf einfache Weise die Regelung des Leistungseintrags bzw. des Energieeintrags unter Berücksichtigung der Gewebeveränderungen oder zu erwartender Gewebeveränderungen.

Vorzugsweise ist der Messgewebebereich kleiner als der Zielgewebebereich ausgebildet, so dass die zum 'Vermessen' des Messgewebebereichs erforderlichen Bestandteile der Messvorrichtung unabhängig von den Ausmaßen des Zielgewebebereichs in dem zu behandelnden Gewebe positioniert werden können. Die Ermittlung von Verlusten, die sich im Zielgewebebereich ergeben würden, könnten dann mit oben beschriebenen Erfahrungswerten festgestellt werden. Ist der Messgewebebereich mit dem Zielgewebebereich identisch, wird also unmittelbar der Zielgewebebereich vermessen, sind die Messwert aufnehmenden Bestandteile der Messvorrichtung exakt im Zielgewebebereich zu platzieren. Dies erfordert die Kenntnis der genauen Ausdehnung des Zielgewebebereichs, die z. B. über bildgebende Verfahren ermittelt werden kann.

Vorzugsweise umfasst die Messvorrichtung mindestens eine in einem definierten Abstand zur Koagulationselektrode angeordnete Messelektrode, die mit dem zu behandelnden Gewebe im Messgewebebereich in elektrisch leitenden Kontakt bringbar ist, sowie mindestens eine Spannungsmesseinrichtung zur Messung einer, unabhängig von etwaigen Leitungsverlusten oder dergleichen Verlusten, über dem Messgewebebereich abfallenden Spannung. So lässt sich z. B. eine in den Messgewebebereich eingebrachte Leistung aus den gemessenen Werten durch die Recheneinrichtung ermitteln, um daraus wieder auf den Endwert für den Zielgewebeenergieeintrag zu schließen.

Die Messelektrode dient als Kontaktfläche für das zu behandelnde Gewebe, definiert somit, zusammen mit der Koagulationselektrode den Messgewebebereich, zumindest in einer Richtung, und liegt bereits im Strompfad hin zur Neutralelektrode. Zwischen Koagulationselektrode und Messelektrode ist die Spannungsmesseinrichtung derart geschaltet, dass der Spannungsabfall über diesem Messgewebebereich ermittelbar ist. Die Kontaktfläche ist also über eine Messleitung mit der Spannungsmesseinrichtung zwischen diesem Messpunkt und der Koagulationselektrode, also der stromeinspeisenden, aktiven Elektrode verbunden. Das heißt, zumindest der so erfassbare Spannungsabfall im Messgewebebereich dient somit als Grundlage zur Feststellung des Messgewebeenergieeintrags in den Messgewebebereich und ggf. zur Ermittlung des notwendigen Energieeintrags in den Zielgewebebereich, indem auf Basis des gemessenen Wertes auf den erforderlichen Energieeintrag in den Zielgewebebereich 'hochgerechnet' wird. Der Messgewebeenergieeintrag kann aber auch einen bloßen Hinweis darauf geben, welche Energie noch in den Zielgewebebereich eingetragen werden muss, um den notwendigen Energieeintrag zu erreichen. Das heißt, der gemessene Wert bzw. der ermittelte Messgewebeenergieeintrag dienen beispielsweise als Vergleichswert. Der Wert des erforderlichen Energieeintrags in den Zielgewebebereich wird dann z. B. 'von außen' vorgegeben, wobei der Vergleichswert aufzeigt, wie viel Energie noch in den Zielgewebebereich eingetragen werden muss.

Bei dieser Ausführungsform lässt sich der durch das zu behandelnde Gewebe, also der durch den definierten Zielgewebebereich bestimmte Widerstand in einem Ersatzschaltbild als in Reihe mit dem oben beschriebenen Übergangswiderstand zwischen Koagulationselektrode und Zielgewebebereich und mit dem Patientenwiderstand geschaltet verstehen. Nachdem vorzugsweise im Messgewebebereich gemessen wird, der kleiner als der Zielgewebebereich ist, teilt sich der Widerstand des definierten Zielgewebebereichs auf in einen Widerstand des Messgewebebereichs und einen Restwiderstand, der aus dem übrigen Zielgewebebereich resultiert. Die Ausführungsform sieht vor, dass lediglich der durch den Koagulationsvorgang bedingte Spannungsabfall der Quellenspannung über dem Messgewebebereich erfasst wird, um daraus auf die eingebrachte Leistung und schließlich auf den erforderlichen Energieeintrag in den Zielgewebebereich zu schließen, was ein besonders einfaches Messverfahren darstellt. Dies ist insofern möglich, als - wie bereits oben beschrieben - der entsprechende Strom im Chirurgiegerät erfassbar ist. Allerdings ist meist auch der Übergangswiderstand zu berücksichtigen. Ist der Übergangswiderstand entsprechend hoch und damit nicht vernachlässigbar, so muss in die Ermittlung des Endwertes für den Zielgewebeenergieeintrag zur Korrektur der durch den Übergangswiderstand bedingte Spannungsabfall einfließen.

Wenn sichergestellt ist, dass beispielsweise durch Kühlung der Elektrode, durch kontaktvermittelnde, aus der Elektrode in das Gewebe strömende Fluide oder durch Anpassung der Elektrodenform (Anpressung) an das Gewebe, der Übergangswiderstand zwischen der Elektrode und dem Messgewebebereich gegenüber dem Messgewebewiderstand klein bleibt, so kann die Messung der Spannung über dem Messgewebebereich auf die Messung des Spitzenwertes beschränkt und auf die Ermittlung des Endwertes für den Energieeintrag verzichtet werden. Dabei wird ausgenutzt, dass mit fortschreitender Koagulation des definierten Messgewebebereichs dessen Widerstand ansteigt. Die Beschränkung auf die Messung des Spitzenwertes hat den Vorteil, dass der Aufwand für die Messeinrichtung insbesondere bei Verwendung nicht sinusförmiger Koagulationsspannungen erheblich vereinfacht wird.

Zur Messung des Spannungsabfalls über dem Messgewebebereich bzw. dem Zielgewebebereich ist es alternativ möglich, einen gesonderten Messstrom, beispielsweise über einen zusätzlichen Schaltkreis (z. B. einen Multiplexer) in den Messgewebebereich zu leiten, so dass ein durch diesen Messstrom verursachter Spannungsabfall über dem Messgewebebereich als Maß für die Impedanz des Messgewebes (und damit des Zielgewebes) messbar ist.

Vorzugsweise weist die Messvorrichtung ferner mindestens eine Strommesseinrichtung zur Messung des HF-Stromes in dem Messgewebebereich zur Ermittlung des Endwertes für den Zielgewebeenergieeintrag durch die Recheneinrichtung auf. Wie bereits oben beschrieben, entspricht der Strom durch den Messgewebebereich dem Generatorstrom und kann mit herkömmlicher Technik im Chirurgiegerät erfasst werden. Zur Vermessung des Messgewebebereichs bietet sich ggf. eine zusätzliche Strommesseinrichtung an, um die Erfassung des in dem Messgewebebereich auftretenden Stromes zu erleichtern und fehlerhafte Berechnungen durch Verlustströme auszuschließen. Weiterhin können Leitungsverluste insbesondere durch kapazitive Kopplung zwischen den Leitungen durch die Bestimmung der Phasenverschiebung herausgerechnet werden.

In einer bevorzugten Ausführungsform weist die Messvorrichtung ferner mindestens eine erste Zeitmesseinrichtung zur Messung einer Dauer des Stromflusses in den Messgewebebereich zur Ermittlung des Endwertes für den Zielgewebeenergieeintrag durch die Recheneinrichtung auf. Ferner ist es möglich, dass die Messvorrichtung mindestens eine zweite Zeitmesseinrichtung zur Messung einer Dauer des Messgewebeenergieeintrags in den Messgewebebereich aufweist, so dass der Endwert für den Zielgewebeenergieeintrag in Abhängigkeit von der Dauer des Messgewebeenergieeintrags durch die Recheneinrichtung ermittelbar ist. Wie bereits oben beschrieben kann es vorteilhaft sein, auch die Zeit, die zur Applikation der Leistung nötig ist, zu ermitteln, insbesondere dann, wenn nicht auf eine bloße Bestimmung einer eingebrachten Arbeit abgestellt werden soll. So kann sich beispielsweise ein hoher Leistungseintrag in den Mess- und damit in den Zielgewebebereich über eine kürzere Zeitdauer auf den Koagulationsgrad des Gewebes ggf. anders auswirken, als ein niedrigerer Leistungseintrag über eine längere Zeitspanne, auch wenn bei beiden Leistungseinträgen die gleiche Arbeit verrichtet wird. Ein hoher Leistungseintrag über eine kurze Zeitdauer bewirkt möglicherweise einen zu starken Koagulationsgrad und damit eine unnötige Schädigung des zu behandelnden Gewebes oder gar des umliegenden Gewebes. Umgekehrt kann ein zu niedriger Leistungseintrag permanent einen ausreichenden Koagulationsgrad verhindern, auch wenn der Leistungseintrag über eine lange Zeitdauer stattfindet. Durch die Erfassung der Zeit, in der der Leistungseintrag stattfindet, kann daher der Koagulationsverlauf besser kalkuliert werden.

Gegebenenfalls bietet es sich an, die Koagulation intervallartig durchzuführen, wie dies beispielsweise bei einem Auftauvorgang mit Hilfe einer Mikrowelle vorgesehen ist. Das Gewebe wird also in kurzen Abständen erwärmt und überschüssige Wärme wird während der Unterbrechung des Koagulationsvorganges für ein weiteres Koagulieren genutzt.

Wie bereits oben erwähnt, lässt sich der Endwert für den Zielgewebeenergieeintrag in den Zielgewebebereich z. B. durch Extrapolation oder durch mit Erfahrungswerten unterstützte Extrapolation bestimmen. Erfahrungswerte werden auch benötigt, um von dem Messgewebebereich auf den Zielgewebebereich hinsichtlich möglicher Energieverluste schließen zu können. Die hierfür benötigten Erfahrungswerte liegen vorzugsweise für dem zu behandelnden Gewebe ähnliche Gewebearten bereits vor. Der Messvorrichtung ist daher eine Speichereinrichtung zur Speicherung experimentell ermittelter Messreihen von Vergleichsprobengewebe beschreibenden Messwerten zugeordnet, so dass für die Ermittlung des Endwertes des Zielgewebeenergieeintrags durch die Recheneinrichtung die gespeicherten Messreihen zugrunde legbar sind. Folglich kann auf einfache Weise auf die experimentell gewonnenen Erfahrungswerte von Gewebeeigenschaften zurückgegriffen werden, die letzten Endes das Koagulationsverhalten bestimmter Gewebearten wiedergeben. So lassen beispielsweise Wärmekapazitäten oder Wärmeleitfähigkeiten unter Berücksichtigung der Gewebeausmaße Rückschlüsse auf das Koagulationsverhalten des zu behandelnden Gewebes zu. Vorzugsweise sollten für entsprechende Eingriffe Messreihen verschiedenster Gewebearten zur Verfügung stehen, die z. B. in dem hier beschriebenen HF-Chirurgiegerät speicherbar sind. Damit können die experimentell gewonnenen Daten während eines Eingriffs in das Berechnungs- und Ermittlungsprozedere der oben beschriebenen Messvorrichtung und Recheneinrichtung einfließen. Die so genutzten Erfahrungswerte bieten für die Ermittlung des Endwertes eine höhere Präzision für den Koagulationsvorgang, als dies durch Verwendung der eingangs beschriebenen Erfahrungswerte, ohne Berücksichtigung der Messungen der Fall wäre.

Der Steuerungseinrichtung ist vorzugsweise eine weitere Messvorrichtung zugeordnet, welche derart ausgebildet ist, dass das zu behandelnde Gewebe beschreibende Messwerte zu deren Aufnahme als Vergleichswerte in die Speichereinrichtung messbar sind. Damit lassen sich diese Messwerte aus dem zu behandelnden Gewebe vor oder auch während einer Operation, ggf. auch danach, aufnehmen und abspeichern und stehen auf diese Weise für weitere Eingriffe zur Verfügung. Zudem kann das zu behandelnde Gewebe für die oben beschriebene Zeitplanregelung unmittelbar genutzt werden, so dass sich neue Endwerte für den Zielgewebeenergieeintrag (Sollwerte) aus sich während der Koagulation verändernden Gewebeparametern ergeben. Um die Aufnahme der Gewebeparameter zu ermöglichen, ist der Steuerungseinrichtung eine Eingabeeinheit zugeordnet, welche derart ausgebildet ist, dass ein Anwender die ein Vergleichsprobengewebe beschreibenden Messwerte und/oder bekannte Gewebeparameter zur Speicherung in die Speichereinrichtung eingeben kann. Vorzugsweise ist die oben beschriebene weitere Messvorrichtung derart ausgebildet, dass die an dem zu behandelnden Gewebe erfassten Messwerte selbsttätig in die Speichervorrichtung aufgenommen werden.

Für eine Nachverfolgung der in den Messgewebebereich eingetragenen Wärmemenge kann sich die Beobachtung einer Temperaturänderung in dem Messgewebebereich als nützlich erweisen. Die Messvorrichtung weist dazu ferner mindestens eine Temperaturmesseinrichtung zur Messung einer Gewebetemperatur in dem Messgewebebereich auf. Die Messvorrichtung ist also derart ausgebildet, dass der Endwert für den Zielgewebeenergieeintrag in Abhängigkeit der gemessenen Temperatur oder in Abhängigkeit der gemessenen Temperatur und der gespeicherten Messreihen durch die Recheneinrichtung ermittelbar und/oder korrigierbar ist. Je mehr Daten für die Ermittlung des Endwertes zur Verfügung stehen, desto genauer lässt sich der für die optimale Koagulation erforderliche Endwert für den Energieeintrag bestimmen. Insbesondere kann über die Beobachtung der Temperatur beispielsweise auf einen geeigneten bzw. ungeeigneten Leistungseintrag geschlossen werden und auch darauf, inwieweit das Gewebe eine für die Devitalisierung ausreichende Temperatur erreicht hat. Bei Verwendung zweier Temperaturmesseinrichtungen ist es möglich, ein Temperaturgefälle im Messgewebebereich zu erfassen, um so den Koagulationsvorgang noch exakter nachvollziehen zu können.

Ferner ist es möglich, auf 'Verhältnisse' an der Koagulationselektrode zu schließen, beispielsweise darauf, ob die Elektrode noch ausreichend frei von Geweberückständen oder dergleichen Verunreinigungen ist. Nur so wäre nämlich eine ausreichende Stromeinbringung in das zu behandelnde Gewebe überhaupt noch gewährleistet. Eine abfallende Temperatur im Messgewebebereich würde also darauf schließen lassen, dass möglicherweise die Stromeinbringung trotz aktiviertem HF-Generator reduziert ist.

Wenn ein Anstieg des Übergangswiderstandes zwischen Koagulationselektrode und Messgewebebereich bei zunehmendem Koagulationsgrad nicht verhindert werden kann (beispielsweise durch oben beschriebene Maßnahmen, wie Anpassung der Elektrodenform an das Gewebe), so muss dieser bei der Ermittlung des Endwertes für den Zielgewebeenergieeintrag entsprechend berücksichtigt werden. Vorzugsweise weist die Messvorrichtung daher eine Einrichtung zur Messung dieses Übergangswiderstandes auf, so dass ein entsprechender Korrekturwert bei der Ermittlung des Endwertes für den Zielgewebeenergieeintrag einbezogen werden kann.

Soll lediglich die Erfassung des Spannungsabfalls über dem Messgewebebereich als Grundlage für weitere Berechnungen dienen, so sollte diese Methode in der Praxis bevorzugt dann angewendet werden, wenn der Übergangswiderstand zwischen Elektrode und Messgewebe gering ist oder entsprechende Korrekturmaßnahmen vorgenommen werden können. Nur so lässt sich gewährleisten, dass der durch den Messgewebebereich bedingte Spannungsabfall richtig erfasst wird.

Eine bevorzugte Ausführungsform sieht vor, dass die Strommesseinrichtung derart ausgebildet ist, dass der erfasste Strom an die Steuerungseinrichtung übermittelbar ist, so dass diese eine Phasenbeziehung zwischen der Spannung und dem Strom als Korrekturwert ermittelt. Bei höheren Frequenzen nimmt insbesondere der Einfluss kapazitiver Blindwiderstände des biologischen Gewebes zu, aber auch Kapazitäten und Induktivitäten der Zuleitungen machen sich bei hohen Frequenzen bemerkbar. Der Einfluss von Kapazitäten und Induktivitäten bewirkt eine Phasenverschiebung zwischen der HF-Generatorspannung und dem HF-Strom und damit auch zwischen der gemessenen HF-Spannung und dem HF-Strom. Insofern lässt sich über eine Strommessung zusätzlich zu der Spannungsmessung die Phasenbeziehung zwischen Spannung und Strom ermitteln und so eine präzisere Ermittlung des Endwertes für den Zielgewebeenergieeintrag durchführen.

Weiterhin ändert sich die Phasenverschiebung bei sich änderndem Widerstand im Zielgewebebereich durch die Koagulation des Gewebes. Somit können auch (ggf. zusätzlich) durch die Messung der Phasenverschiebung Daten für den hier interessierenden Messwert gewonnen werden.

Die Spannungsmesseinrichtung, aber auch die Strommesseinrichtung sind in einer bevorzugten Ausführungsform als modulare Bausteine der HF-Chirurgieeinrichtung vorgesehen. Damit lassen sich die Messeinrichtungen auf Dauer in das HF-Chirurgiegerät integrieren oder bei Bedarf auch zeitweise entfernen.

Eine bevorzugte Ausführungsform sieht vor, dass die Steuerungseinrichtung derart ausgebildet ist, dass sie das Abschaltsignal an den HF-Generator übermittelt, so dass dieser abschaltet und somit den HF-Strom abschaltet. Damit wird eine besonders einfache und zuverlässige Ausgestaltung zur Deaktivierung der HF-Chirurgieeinrichtung realisiert.

In einer bevorzugten Ausführungsform ist mindestens eine Signalverarbeitungseinrichtung vorgesehen, an die das Abschaltsignal zuführbar ist. Die Signalverarbeitungseinrichtung ist derart ausgebildet, dass mittels des Abschaltsignals eine optische und/oder akustische Anzeige derart ansteuerbar ist, dass das Abschalten des HF-Stromes aufgrund des Abschaltsignals zur Benutzerführung anzeigbar ist. Mit dieser Anzeige wird das Abschalten der HF-Chirurgieeinrichtung und damit die Beendigung-des Koagulationsvorgangs signalisiert. Ein Benutzer kann den Koagulationsvorgang dann z. B. manuell beenden. Grundsätzlich ist es möglich, dass die Anzeige direkt von der Steuerungseinrichtung angesteuert wird.

Die optische Anzeige kann beispielsweise als Display ausgebildet sein, so dass über dieses auf das Abschalten des HF-Stromes hingedeutet wird. Die Signalverarbeitungseinrichtung weist vorzugsweise auch eine Speichereinrichtung auf, die die Abschaltsignale, ggf. samt diverser Randbedingungen früherer chirurgischer Eingriffe abspeichert. Diese Daten können dann über das Display ausgegeben werden. Mittels der angezeigten Erfahrungswerte ist es möglich, vorgefundene Gewebestrukturen hinsichtlich einer anstehenden Behandlung besser beurteilen zu können. Auch die Auswahl eines geeigneten elektrochirurgischen Instruments wird somit erleichtert. Die optische Anzeige kann beispielsweise auch lediglich in Form eine Lampe vorgesehen sein. Durch ein Aufleuchten der Lampe wird das Ende der Koagulation für den Benutzer signalisiert, ggf. auch das Abschalten des HF-Stromes. Eine rein akustische Anzeige weist den Benutzer auf die Beendigung der Koagulation hin, ohne dass dieser eine optische Anzeige verfolgen müsste. Auch eine Kombination von optischer und akustischer Anzeige ist möglich.

Vorteilhafterweise ist die Koagulationselektrode als Kugelelektrode ausgebildet. Diese eignet sich insbesondere für die interstitielle Koagulation, weil die Kugelelektrode eine im Wesentlichen radialsymmetrische Stromdichteverteilung in dem Zielgewebebereich initiiert, insbesondere bei homogenem Gewebe und wenn der Abstand von der Koagulationselektrode zur Neutralelektrode groß ist gegenüber der Ausdehnung des Mess- bzw. Zielgewebebereichs. Somit lässt eine eindimensionale Messung, z. B. des Spannungsabfalls über dem Messgewebebereich, auf die Ausgestaltung der dreidimensionalen Koagulationszone schließen.

Die Koagulationselektrode und die Messelektrode sind derart zueinander angeordnet, dass sie den Messgewebebereich hinsichtlich seiner Ausdehnung zumindest eindimensional in einer Richtung abgreifen. Das heißt, der Abstand zwischen der Koagulationselektrode und der Messelektrode entspricht der Größe des Messgewebebereichs zumindest in dieser einen Richtung. Würde man über den radialsymmetrisch ausgedehnten Messgewebebereich ein dreidimensionales Koordinatensystem legen, so entspräche dessen Nullpunkt einem Angriffspunkt der Koagulationselektrode an dem zu behandelnden Gewebe. Die Koagulationselektrode würde also im Nullpunkt des Koordinatensystems liegen, während die Messelektrode z. B. auf einer der Achsen des Koordinatensystems angeordnet wäre. Damit wird im Wesentlichen der Messgewebebereich in der einen Richtung vermessen, beispielsweise bei Erfassung des Spannungsabfalls über dem entsprechenden Gewebebereich.

Entspricht der Messgewebebereich dem Zielgewebebereich, dann wird die gewünschte Koagulationszone (bei radialsymmetrischer Ausdehnung) zumindest in einer Richtung abgegriffen. Dies ist beispielsweise dann möglich, wenn der Zielgewebebereich z. B. über bildgebende Verfahren vor dem eigentlichen Eingriff exakt festgelegt wird.

In einer bevorzugten Ausführungsform weist die Messvorrichtung zwei von der Koagulationselektrode mit einem definierten Abstand angeordnete Messelektroden auf, die mit dem zu behandelnden Gewebe in elektrisch leitenden Kontakt bringbar sind. Die Messelektroden sind dabei entlang einer Linie mit der Koagulationselektrode an dem elektrochirurgischen Instrument angeordnet, wobei die Koagulationselektrode zwischen den Messelektroden angeordnet ist. In dem hypothetisch vorhandenen dreidimensionalen Koordinatensystem würde die Koagulationselektrode wieder in dessen Nullpunkt angeordnet sein, wobei der Nullpunkt wieder dem Angriffspunkt der Koagulationselektrode an dem Messgewebebereich entspricht. Die erste Messelektrode könnte dann z. B. auf einer positiven Achse, die zweite Elektrode auf der entsprechend negativen liegen. Somit wird der Messgewebebereich eindimensional in zwei Richtungen vermessen. Der Einsatz zweier Messelektroden bietet sich insbesondere bei unsymmetrischen Koagulationszonen an, hervorgerufen z. B. durch nichtkugelförmige Koagulationselektroden oder durch Änderungen der Gewebestruktur innerhalb des Messgewebebereichs. Die Erfassung eines Messwertes in dem Messgewebebereich, z. B. die Erfassung des Spannungsabfalls, erfolgt dann ggf. über zwei Spannungsmesseinrichtungen.

Vorzugsweise ist die Messelektrode an dem elektrochirurgischen Instrument ortsveränderbar angeordnet. Damit lassen sich mit nur einem Instrument unterschiedlich große Messgewebebereich bzw. Zielgewebebereiche vermessen. Die Messelektrode kann dabei verschiebbar und einrastbar ausgebildet sein, oder aber sie ist an unterschiedlichen Stellen in Aufnahmebereiche aufnehmbar.

Ist die Messelektrode an dem elektrochirurgischen Instrument festgelegt, müssten für unterschiedliche Größen der Messgewebebereiche verschiedene elektrochirurgische Instrumente zur Verfügung stehen.

Mit dieser Anordnung ist es nunmehr möglich, auf einfachste Weise den Koagulationsvorgang zu optimieren und die für die Devitalisierung des Gewebes erforderliche Energie auf das benötigte Maß zu reduzieren.

Die vorangehende Beschreibung stellt in erster Linie auf die Erfassung eines Spannungsabfalls über dem Messgewebebereich ab, um so auf den erforderlichen Energieeintrag und damit auf die Größe der Koagulationszone zu schließen. Es ist alternativ jedoch auch möglich, den Widerstand bzw. die Widerstandsänderung des Messgewebebereichs (ggf. auch des Zielgewebebereichs) zu erfassen.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- - Fig. 1: ein funktionales Blockschaltbild, das eine Ausführungsform der erfindungsgemäßen HF-Chirurgieeinrichtung darstellt;
- - Fig. 2: einen Ausschnitt aus dem funktionalen Blockschaltbild gemäß Fig. 1 in vereinfachter Darstellung und
- - Fig. 3: ein Ersatzschaltbild, das die Funktionsweise der Anordnung gemäß der Fig. 1 und 2 mit Hilfe virtueller Bauteile beschreibt.

In der nachfolgenden Beschreibung werden für gleiche und gleich wirkende Teile dieselben Bezugsziffern verwendet.

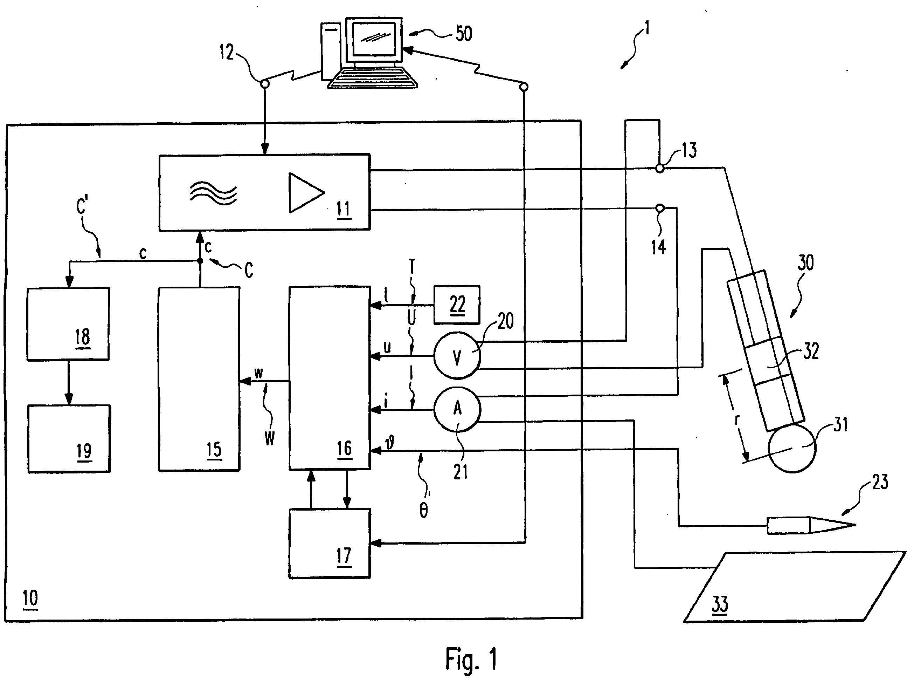

Fig. 1 zeigt eine Ausführungsform der hier offenbarten Vorrichtung. Dabei sind schematisch die für die Erläuterung der Erfindung wesentlichen Komponenten einer HF-Chirurgieeinrichtung 1, nämlich ein HF-Chirurgiegerät 10, ein monopolares elektrochirurgisches Instrument 30 und eine Neutralelektrode 33 gezeigt.

Bei monopolaren Anordnungen, wie in Fig. 1 gezeigt, wird ein von einem HF-Generator 11 an das elektrochirurgische Instrument 30 zugeführter HF-Strom in ein zu behandelndes Gewebe über eine differente Elektrode, hier eine monopolare Koagulationselektrode 31, appliziert, wobei der Strompfad durch den Körper eines Patienten bis zu der indifferenten Neutralelektrode 33 führt.

Das HF-Chirurgiegerät 10 weist einen Eingangsanschluss 12 zum Anschließen von Finger- und/oder Fußschalter aufweisenden Schalteinrichtungen auf. Über diese Schalteinrichtungen wird z. B. ein Aktivieren und/oder Deaktivieren des HF-Stromes ermöglicht. Die Schalteinrichtungen lassen sich hier vorzugsweise über eine Computeranordnung 50 realisieren. Ausgangsseitig sind an dem HF-Chirurgiegerät 10 ein erster Ausgangsanschluss 13 und ein zweiter Ausgangsanschluss 14 vorgesehen, über die das monopolare elektrochirurgische Instrument 30 mit der dazugehörigen Neutralelektrode 33 anschließbar sind.

Gemäß Fig. 1 ist das elektrochirurgische Instrument 30 mit einer kugelförmigen Koagulationselektrode 31 ausgebildet, wobei in einem definierten Abstand r zu der Koagulationselektrode 31 eine mit dem zu behandelnden Gewebe in elektrisch leitenden Kontakt bringbare Messelektrode 32 zum Abgreifen des Messgewebebereichs an dem elektrochirurgischen Instrument 30 angeordnet ist. Die Kugelelektrode 31 ist über den ernsten Ausgangsanschluss 13 mit dem HF-Generator 11 verbunden. Auch die Messelektrode 32 ist mit dem ersten Ausgangsanschluss 13 verbunden, wobei eine Spannungsmesseinrichtung 20 parallel zwischen die Koagulationselektrode 31 und die Messelektrode 32 geschaltet ist. Die Neutralelektrode 33 ist mit dem zweiten Ausgangsanschluss 14 verbunden, wobei in Reihe zwischen die Neutralelektrode 33 und den HF-Generator 11 eine Strommesseinrichtung 21 geschaltet ist. Sowohl die Spannungsmesseinrichtung 20, als auch die Strommesseinrichtung 21 sind hier im HF-Chirurgiegerät 10 integriert ausgebildet. Als modulare Bausteine lassen sich die Messeinrichtungen auf Dauer in das HF-Chirurgiegerät 10 integrieren oder bei Bedarf auch zeitweise entfernen.

Kernstück des HF-Chirurgiegerätes 10 ist der steuerbare HF-Generator 11 zum Erzeugen einer HF-Spannung und zum Zuführen des HF-Stromes zu der Koagulationselektrode 31 des elektrochirurgischen Instruments 30. Die Messelektrode 32, die Spannungsmesseinrichtung 20, die Strommesseinrichtung 21, eine Zeitmesseinrichtung 22 und eine Temperaturmesseinrichtung 23 bilden zusammen mit einer Recheneinrichtung 16 in diesem Ausführungsbeispiel eine Messvorrichtung aus, wobei die Spannungsmesseinrichtung 20 und die Strommesseinrichtung 21 über Steuerungsleitungen U und I und die Temperaturmesseinrichtung 23 über eine Steuerungsleitung θ der Recheneinrichtung 16 zugeordnet sind. Die Zeitmesseinrichtung 22 ist in diesem Ausführungsbeispiel mit dem HF-Generator 11 verbunden und über eine Steuerungsleitung T ebenfalls der Recheneinrichtung 16 zugeordnet. Die Recheneinrichtung 16 ist über eine Steuerungsleitung W mit einer Steuerungseinrichtung 15 verbunden. Auch der HF-Generator 11 ist mit der Steuerungseinrichtung 15 über eine Steuerungsleitung C verbunden. Außerdem ist eine Signalverarbeitungseinrichtung 18 über eine Steuerungsleitung C' mit der Steuerungseinrichtung 15 verbunden, wobei der Signalverarbeitungseinrichtung 18 eine Anzeige 19 zugeordnet ist.

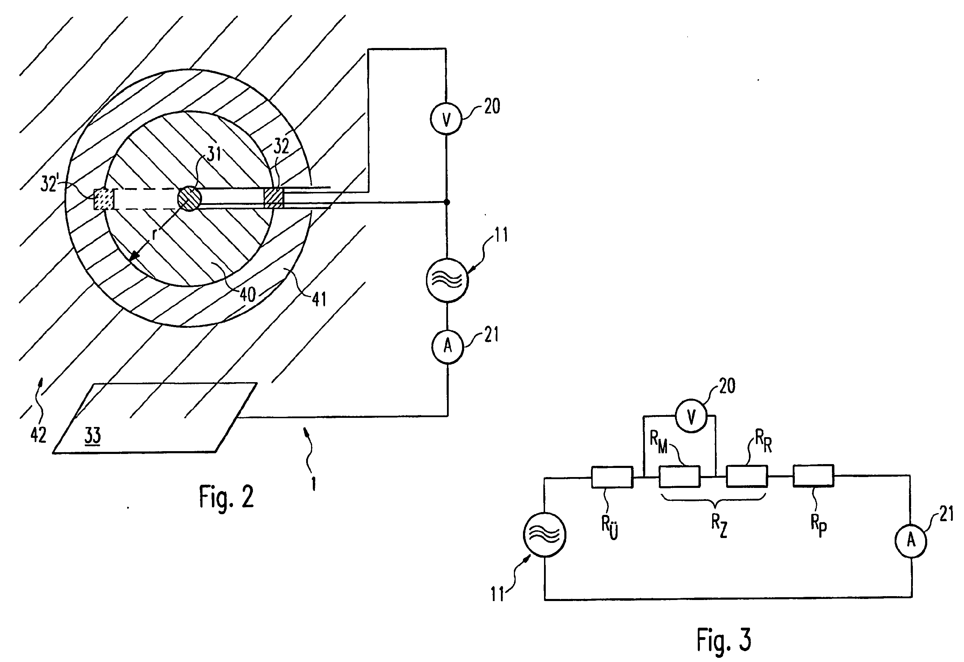

Fig. 2 zeigt einen Ausschnitt aus dem funktionalen Blockschaltbild gemäß Fig. 1 in vereinfachter Darstellung, wobei zusätzlich das zu behandelnde Gewebe abgebildet ist. Das elektrochirurgische Instrument 30 und das zu behandelnde Gewebe sind im Wesentlichen im Schnitt dargestellt. Die Koagulationselektrode 31 ist mit der Messelektrode 32 in das zu behandelnde Gewebe eingeführt, wobei die beiden Elektroden 31 und 32 zumindest in einer Richtung einen Messgewebebereich 40 definieren, der als Teilbereich eines Zielgewebebereichs 41 dargestellt ist. Der Zielgewebebereich 41 und damit auch der Messgewebebereich 40 liegen als das zu behandelnde Gewebe in einem sie umgebenden Gewebe 42, das wiederum einer möglichst geringen Stromeinwirkung ausgesetzt werden soll, jedoch als Strompfad bis hin zur Neutralelektrode 33 dient. Da die Koagulationselektrode 31 in dieser Ausführungsform kugelförmig ausgebildet ist, ergibt sich aufgrund einer im Wesentlichen radialsymmetrischen Stromdichteverteilung eine im Wesentlichen radialsymmetrische Koagulationszone 41, insbesondere bei homogenem Gewebe und wenn der Abstand von der Koagulationselektrode 31 zur Neutralelektrode 33 groß ist gegenüber der Ausdehnung des Mess- bzw. Zielgewebebereichs. Das monopolare elektrochirurgische Instrument 30 mit der Kugelelektrode 31 gemäß Fig. 1 ist z. B. für die interstitielle Devitalisierung von Tumor- oder Metastasengewebe vorgesehen. Beispielsweise bei der Behandlung eines Leber-Tumors wird die Elektrode in den Tumor eingestochen, um das umliegende Gewebe zu koagulieren.

Nachfolgend wird die Funktionsweise der hier offenbarten HF-Chirurgieeinrichtung 1 beschrieben.

Die Koagulationselektrode 31 ist zusammen mit der Messelektrode 32 in das zu behandelnde Gewebe eingebracht. Über die Koagulationselektrode 31 wird der hochfrequente Strom in das zu behandelnde Gewebe zugeführt. Die Stromdichte verteilt sich in diesem Falle im Wesentlichen radialsymmetrisch im Gewebe, da hier eine Kugelelektrode eingesetzt wird, und erfasst dabei auch die Messelektrode 32. Die Messvorrichtung ist dazu ausgebildet, dass sie mindestens einen einen über die Koagulationselektrode 31 bewirkten Energieeintrag in den Messgewebebereich 40 beschreibenden Messwert u, i, t, ϑ erfasst, um daraus auf mögliche Energieverluste zu schließen, die einen Gesamtenergieeintrag in das zu behandelnde Gewebe schmälern. Der zu erfassende Messwert kann z. B. als ein über dem Messgewebebereich 40 messbarer Spannungsabfall u erfasst werden, wobei der Spannungsabfall in dem zwischen der Koagulationselektrode 31 und der Messelektrode 32 liegenden Gewebe mit Hilfe der Messelektrode 32 und der mittels einer Messleitung parallel zwischen die Elektroden 31 und 32 geschalteten Spannungsmesseinrichtung 20 erfassbar ist. Die Messelektrode 32 dient also als Kontaktfläche für das zu behandelnde Gewebe, grenzt somit den Messgewebebereich 40 ab und liegt bereits im Strompfad hin zur Neutralelektrode 33. Wenn der Strom i durch den Messgewebebereich 40, wie bereits oben beschrieben, mit herkömmlicher Technik im Chirurgiegerät erfasst werden kann, lässt sich über den Spannungsabfall u z. B. der Leistungseintrag in den Messgewebebereich 40 ermitteln. Das heißt, mittels der Messelektrode 32 wird der Spannungsabfall u im Messgewebebereich 40 gemessen (und über die Spannungsmesseinrichtung 20 angezeigt), wobei die Recheneinrichtung 16 daraus zusammen mit dem entsprechenden Stromwert z. B. den entsprechenden Leistungseintrag feststellt. Die Erfassung des Spannungsabfalls u über nur einem ausgewählten Bereich des Messgewebebereiches ist aufgrund der radialsymmetrischen Ausdehnung des Zielgewebebereichs 41 möglich. Über den Leistungseintrag wiederum kann dann z. B. auf den für den Zielgewebebereich erforderlichen Energieeintrag geschlossen werden.

Es wird also über einen für den Messgewebeenergieeintrag charakteristischer Messwert - hier des Spannungsabfalls u - schließlich ein Energieeintrag festgestellt, um darauf und/oder auf Erfahrungswerte beruhend, den HF-Generator derart zu steuern oder zu regeln, dass der für das optimale Koagulationsergebnis erforderliche Energieeintrag in den Zielgewebebereich durch ein Angleichen des erfolgten Energieeintrags an den vorgegebenen, ggf. ermittelten Endwert für den Zielgewebeenergieeintrag in den Zielgewebebereich erreicht wird.

Die Feststellung des Energieeintrags in den Messgewebebereich ist also erforderlich, weil nicht vermeidbare Energieverluste, beispielsweise thermische Verluste, über den Messgewebebereich bzw. den Zielgewebebereich umgebende Gewebebereiche (letztendlich bis zu einer indifferenten Elektrode hin) oder auch eine elektrische Verlustleistung, verursacht z. B. durch vagabundierende Ströme, kaum erfassbar sind. Der von der Koagulationselektrode eingebrachte Gesamtenergieeintrag steht nämlich aufgrund dieser Verluste nicht vollständig für den Zielgewebebereich zur Verfügung. Die hier aufgezeigte Messvorrichtung stellt nun darauf ab, mindestens den Messgewebebereich zu 'vermessen', so dass letztendlich beurteilt werden kann, wie viel Energieeintrag für den Zielgewebebereich erhalten bleiben wird und für ein optimales Koagulationsergebnis z. B. noch nötig ist.

Fig. 3 zeigt ein Ersatzschaltbild, das den Strompfad bei der Behandlung eines Patienten mittels der monopolaren Koagulation und die im Strompfad liegenden Widerstände verdeutlicht, die neben dem Zielgewebebereich 41 die oben beschriebenen Verluste verursachen. Ein Widerstand R_{Z} des Zielgewebebereichs 41 setzt sich zusammen aus einem Widerstand R_{M} des Messgewebebereichs und einem Restwiderstand R_{R} des übrigen Zielgewebebereichs. Die Widerstände R_{M} und R_{Z} sind als in Reihe mit einem Patientenwiderstand R_{P} geschaltet zu verstehen. Der Begriff 'Patientenwiderstand' meint hier das nicht zu behandelnde Gewebe, das zwischen dem Zielgewebebereich und der Neutralelektrode als Strompfad zur Verfügung steht, sowie ggf. einen weiteren Übergangswiderstand zwischen diesem Gewebe und der am Patienten angebrachten Neutralelektrode. Unter ungünstigen Koagulationsbedingungen ist bei der Ermittlung des Endwertes w für den Zielgewebeenergieeintrag auch ein Übergangswiderstand R_{Ü} zwischen der Koagulationselektrode 31 und dem zu behandelnden Gewebe zu berücksichtigen, der für weitere Verluste aus dem Gesamtenergieeintrag verantwortlich ist. Dazu weist das HF-Chirurgiegerät 10 vorzugsweise eine hier nicht gezeigte Einrichtung zur Erfassung des Übergangswiderstandes R_{Ü} auf.

Die Erfassung des Messwertes in dem expliziten Messgewebebereich 40 (statt unmittelbar in dem Zielgewebebereich 41) dient der Vereinfachung der Messung. Die Elektroden 31, 32 können so nämlich unabhängig von Ausmaßen des Zielgewebebereichs 41 in das zu behandelnde Gewebe eingeführt werden, d. h., Koagulationselektrode 31 und Messelektrode 32 müssen nicht derart zueinander beabstandet angeordnet werden, dass sie exakt den definierten Zielgewebebereich 41 erfassen. In einem Sonderfall entspricht der 'zu vermessende' Messgewebebereich 40 jedoch dem Zielgewebebereich 41, so dass der Energieeintrag ebenfalls dem Zielgewebeenergieeintrag entspricht.

Auf Basis des mindestens einen Messwertes u (und der damit erfassten Verluste) und z. B. anhand vorliegender Erfahrungswerte soll dann ein für ein optimales Koagulationsergebnis erforderlicher Sollwert bzw. Endwert w für den Zielgewebeenergieeintrag in den Zielgewebebereich 41 ermitteln werden. Die Recheneinrichtung 16 ist z. B. dazu ausgelegt, diesen Endwert w zu ermitteln. Das heißt, die Messvorrichtung ist immer derart ausgebildet, dass sie die erforderlichen Messwerte zur Berechnung bzw. Ermittlung des erfolgten und erforderlichen Energieeintrags, ggf. auf Erfahrungswerte beruhend, durch die Recheneinrichtung dieser zur Verfügung stellt. Der Endwert w für den Zielgewebeenergieeintrag ist im Prinzip vorgegeben, da er beispielsweise auf Erfahrungswerte beruht oder aus aktuellen Gewebeparametern ermittelt wird. Grundsätzlich ließe sich der Endwert für den Zielgewebeenergieeintrag aus dem im Messgewebebereich gemessenen Wert, hier der Spannung u und dem bekannten Strom i, z. B. durch Extrapolation, ermitteln. Dies wäre insbesondere dann möglich, wenn der Messgewebebereich dem Zielgewebebereich entspricht. Zur Präzisierung einer Stromregelung bietet es sich in der Praxis jedoch an, die Ermittlung des Endwertes w zumindest zusätzlich unter Bezugnahme auf Erfahrungswerte durchzuführen. Die Erfahrungswerte liegen beispielsweise aus früheren Eingriffen vor und berücksichtigen bekannte Gewebeparameter. Damit lassen sich Endwerte w als Sollwerte vorgeben, an die der Energieeintrag in den Messgewebebereich 40 als momentan vorliegender Istwert über die Stromregelung (-steuerung) anzugleichen ist, wobei der Messgewebeenergieeintrag mit einem Istwert der gemessenen Größe (woraus sich der Istwert des Messgewebeenergieeintrags ergibt) in funktionellem Zusammenhang steht. Letzteres ist insbesondere dann empfehlenswert, wenn sich der Messgewebebereich 40 vom Zielgewebebereich 41 unterscheidet.

Auf Grundlage des ermittelten und/oder vorgegebenen Endwertes w wird der erforderliche HF-Strom an das zu behandelnde Gewebe über die Koagulationselektrode 31 zugeführt und zwar so lange, bis der optimale Energieeintrag in das zu behandelnde Gewebe, d. h. der Endwert w, erreicht ist. Die Steuerungseinrichtung 15 erzeugt ein Abschaltsignal c, wenn der Endwert w erreicht ist, das anzeigt, dass der Koagulationsvorgang zu einem optimalen Zeitpunkt beendet ist. Im einfachsten Fall wird das Abschaltsignal erzeugt, wenn der gemessene Wert, also z. B. der Spannungsabfall, einen bestimmten - auf Erfahrungswerten beruhenden - Wert erreicht hat. Am einfachsten wird der HF-Generator 11 derart von der Steuerungseinrichtung 15 angesteuert, dass er aufgrund des Abschaltsignals c abschaltet. Die Erfassung bzw. Ermittlung des für eine optimale Koagulation erforderlichen Endwertes w für den Energieeintrag in den Zielgewebebereich 41, hier über den Messgewebebereich 40, ermöglicht eine präzise Behandlung des definierten Gewebes, wobei das umgebende Gewebe 42 maximal geschont wird.

Die Recheneinrichtung 16 ist vorzugsweise derart ausgebildet, dass der erforderliche Endwert w in zeitlich definierten Abständen wiederholt neu ermittelt wird. Der Endwert w wird so entsprechend einem festen zeitlichen Ablauf einer Zeitplanregelung vorgegeben. Die Zeitplanregelung ist z. B. dann erforderlich, wenn der zu behandelnde Gewebebereich während des Koagulationsvorganges beobachtet und dessen Gewebeveränderungen berücksichtigt werden sollen bzw. wenn aus der Erfahrung bereits bekannt ist, dass der Sollwert einer ständigen Anpassung während der Koagulation bedarf. Die sich während der Koagulation verändernden Gewebeparameter bilden somit die Basis für den weiteren Koagulationsverlauf. Das heißt, der optimale Endwert w für den Zielgewebeenergieeintrag kann sich im Laufe der Koagulation derart verändern, dass eine wiederholte Neubestimmung des Endwertes, dem der Istwert folgen soll, erforderlich ist.

Die Zeitplanregelung kann alternativ derart vorgesehen sein, dass eine feste Zeitplanregelung vorab, beispielsweise auf Erfahrungswerte beruhend, vorgegeben wird, wobei der Koagulationsverlauf in dem zu behandelnden Gewebe z. B. unberücksichtigt bleibt. Auch bietet sich eine Kombination aktueller Gewebeparameter mit vorgegebenen Erfahrungswerten an, um einen optimalen Energieeintrag in das zu behandelnde Gewebe, also in den Zielgewebebereich, zu erzielen.

Eine Zeitplanregelung ermöglicht auf einfache Weise die Regelung des Leistungseintrags und damit des Energieeintrags unter Berücksichtigung der Gewebeveränderungen oder zu erwartender Gewebeveränderungen.

Soll nicht nur die in das Gewebe eingebrachte Leistung ermittelt werden, sondern auch eine Zeitdauer t, über die der Leistungseintrag stattfindet, so kann die Zeitdauer t mit Hilfe der Zeitmesseinrichtung 22 erfasst werden. Im einfachsten Falle erfasst die Zeitmesseinrichtung 22 jeweilige Aktivierungsphasen des HF-Generators 11, also beispielsweise die Dauer der Stromzuführung in das zu behandelnde Gewebe. Damit ist festgelegt, wie lange der HF-Strom dem zu behandelnden Gewebe zugeführt wurde, so dass sich der Messgewebeenergieeintrag in den Messgewebebereich 40 als über die Zeit eingebrachte Leistung und/oder Arbeit indirekt aus den gemessenen Werten (Strom, Spannung und Zeit) feststellen lässt (im Falle der thermischen Devitalisierung durch den HF-Strom entspricht die Energiemenge der über die Zeit integrierten im Mess- bzw. Zielgewebebereich umgesetzten elektrischen Leistung). Dazu führt die Zeitmesseinrichtung 22 über die Steuerungsleitung T die entsprechende Zeitdauer t der Recheneinrichtung 16 zu. Die Erfassung des zeitlichen Verlaufs des Energieeintrags ist insofern auch vorteilhaft, als damit unterschiedliche Leistungseinträge mit unterschiedlichen Zeitverläufen berücksichtigt werden, auch wenn durch diese ggf. die gleiche Arbeit verrichtet wird. Es wird also auf die Höhe der Leistung in Abhängigkeit der Dauer des Eintrags abgestellt, so dass sich der Koagulationsverlauf besser kalkulieren lässt.

Für eine Nachverfolgung der in den Messgewebebereich 40 eingetragenen Wärmemenge kann sich die Beobachtung einer Temperaturänderung in dem Messgewebebereich 40 als nützlich erweisen. Die Messvorrichtung weist dazu die Temperaturmesseinrichtung 23 zur Messung einer Gewebetemperatur ϑ in dem Messgewebebereich auf, die über die Steuerleitung θ an die Recheneinrichtung 16 übermittelt wird. Dabei ist die Messvorrichtung derart ausgebildet, dass der Messgewebeenergieeintrag in Abhängigkeit der gemessenen Temperatur ϑ feststellbar und/oder korrigierbar ist. Insbesondere kann über die Beobachtung der Temperatur beispielsweise auf einen geeigneten bzw. ungeeigneten Leistungseintrag bzw. Energieeintrag geschlossen werden und auch darauf, inwieweit das Gewebe eine für die Devitalisierung ausreichende Temperatur erreicht hat. Diese Daten erleichtern und präzisieren die Ermittlung des Endwertes für den Zielgewebeenergieeintrag. Vorteilhaft ist die Verwendung von mindestens zwei Temperaturmesseinrichtungen, um ein Temperaturgefälle in dem zu behandelnden Gewebe nachvollziehen zu können.

Wie bereits erwähnt, lässt sich der Endwert w für den Zielgewebeenergieeintrag in den Zielgewebebereich z. B. durch Extrapolation oder durch mit Erfahrungswerten unterstützte Extrapolation bestimmen. Erfahrungswerte werden auch benötigt, um von dem Messgewebebereich auf den Zielgewebebereich hinsichtlich möglicher Energieverluste schließen zu können. Der Messvorrichtung ist daher die Speichereinrichtung 17 zur Speicherung von experimentell ermittelten Messreihen von Vergleichsprobengewebe beschreibenden Messwerten zugeordnet. Für die Ermittlung des Endwertes w werden dann neben einer allgemeinen Extrapolation die gespeicherten Messreihen zugrunde gelegt. So lassen beispielsweise Wärmekapazitäten oder Wärmeleitfähigkeiten Rückschlüsse auf das Koagulationsverhalten des zu behandelnden Gewebes zu.

Eine weitere (hier nicht gezeigte) Messvorrichtung, die ebenfalls der Steuerungseinrichtung 15 zugeordnet ist, ermöglicht alternativ die Vermessung des zu behandelnden Gewebes. Damit können die für das zu behandelnde Gewebe charakteristischen Messwerte unmittelbar in die Speichereintichtung 17 aufgenommen werden. Zudem kann das zu behandelnde Gewebe für die oben beschriebene Zeitplanregelung unmittelbar genutzt werden, so dass sich neue Endwerte w für den Zielgewebeenergieeintrag (Sollwerte) aus sich während der Koagulation verändernden Gewebeparametern ergeben. Um die Aufnahme der Gewebeparameter zu ermöglichen, ist der Speichereinrichtung 17 ebenfalls die Eingabeeinheit 50 zugeordnet, welche derart ausgebildet ist, dass ein Anwender die ein Vergleichsprobengewebe beschreibenden Messwerte und/oder bekannte Gewebeparameter zur Speicherung in die Speichereinrichtung 17 eingeben kann. Vorzugsweise ist die oben beschriebene weitere Messvorrichtung derart ausgebildet, dass die an dem zu behandelnden Gewebe erfassten Messwerte selbsttätig in die Speichervorrichtung 17 aufgenommen werden.

Bei einer kugelförmigen Elektrode und damit einer radialsymmetrischen Koagulationszone 41 lässt eine eindimensionale Messung (Koagulationselektrode 31 mit beabstandeter Messelektrode 32), insbesondere des Spannungsabfalls über dem Messgewebebereich 40, auf die Ausgestaltung der dreidimensionalen Koagulationszone 41 schließen. Bei unsymmetrischen Koagulationszonen, wie sie beispielsweise durch nichtkugelförmige Koagulationselektroden entstehen können, sollten zur sicheren Erkennung bzw. Bestimmung mindestens einer Dimension der Koagulationszone 41, in zwei entgegengesetzten Richtungen, ausgehend von der Koagulationselektrode 31, mindestens zwei Messelektroden 32, 32' an dem elektrochirurgischen Instrument 30 angeordnet sein. Die Messelektroden 32, 32' und die Koagulationselektrode 31 sind dazu, wie in Fig. 2 gezeigt, entlang einer Linie ausgebildet, wobei die Koagulationselektrode 31 zwischen den Messelektroden 32, 32' angeordnet ist. Die Erfassung des Messwertes, hier des Spannungsabfalls u über dem Messgewebebereich 40 erfolgt dann ggf. über zwei Spannungsmesseinrichtungen. Vorzugsweise ist die Messelektrode 32, 32' an dem elektrochirurgischen Instrument 30 ortsveränderbar angeordnet. Damit lassen sich mit nur einem Instrument unterschiedlich große Messgewebebereiche 40 bzw. Zielgewebebereiche 41 vermessen. Die Messelektrode 32, 32' kann dabei verschiebbar und einrastbar ausgebildet sein, oder aber sie ist an unterschiedlichen Stellen in Aufnahmebereiche aufnehmbar.

Ist die Messelektrode 32, 32' an dem elektrochirurgischen Instrument 30 festgelegt, müssten für unterschiedliche Größen der Messgewebebereiche verschiedene elektrochirurgische Instrumente zur Verfügung stehen.

Wie in den Fig. 1 bis 3 gezeigt, weist die HF-Chirurgieeinrichtung 1 die oben bereits beschriebene Strommesseinrichtung 21 auf. Die Messung des HF-Stromes ermöglicht die Ermittlung einer Phasenbeziehung zwischen Strom und Spannung, hier beispielsweise mit Hilfe der Steuerungseinrichtung 15. Bei höheren Frequenzen nimmt insbesondere der Einfluss kapazitiver Blindwiderstände des biologischen Gewebes zu, aber auch Kapazitäten und Induktivitäten der Zuleitungen machen sich bei hohen Frequenzen bemerkbar. Insofern lässt sich über die Ermittlung der Phasenbeziehung zwischen Spannung und Strom eine präzisere Ermittlung des Endwertes w für den Zielgewebeenergieeintrag durchführen.

Gemäß Fig. 1 weist das HF-Chirurgiegerät 10 die oben bereits erwähnte Signalverarbeitungseinrichtung 18 auf, an die das Abschaltsignal c über die Steuerleitung C' zuführbar ist. Die Signalverarbeitungseinrichtung 18 ist derart ausgebildet, dass sie das Abschaltsignal c der optischen und/oder akustischen Anzeige 19 übermittelt, so dass die Abschaltung des HF-Stromes aufgrund des Abschaltsignals c zur Benutzerführung anzeigbar ist. Eine rein akustische Anzeige weist den Benutzer auf die Beendigung der Koagulation hin, ohne dass dieser eine optische Anzeige verfolgen müsste. Auch eine Kombination von optischer und akustischer Anzeige ist möglich.

Die optische Anzeige kann als Display und/oder beispielsweise als Lampe ausgebildet sein. Die Signalverarbeitungseinrichtung 18 weist vorzugsweise eine Speichereinrichtung (nicht gezeigt) auf, die die Abschaltsignale, ggf. samt diverser Randbedingungen, früherer chirurgischer Eingriffe abspeichert. Die Daten sind über das Display 19 anzeigbar, so dass der Operateur für einen anstehenden Eingriff Erfahrungswerte der früheren Behandlungen nutzen kann.

Wie bereits beschrieben, kann als der zu erfassende Messwert die über dem Messgewebebereich 40 abfallende Spannung vorgesehen sein. Es ist jedoch auch möglich, den Widerstand bzw. die Widerstandsänderung des Messgewebebereichs 40 (ggf. des Zielgewebebereichs 41) während dessen Koagulation zu erfassen, um daraus einen Zusammenhang mit der zu erwartenden Größe der Koagulationszone zu ermitteln.

Die Erfindung ist sowohl für Koagulations- als auch für Schneidvorgänge anwendbar. Insbesondere kann es von Vorteil sein, die mit den Schneidvorgängen auftretende Koagulationswirkung erfindungsgemäß zu erfassen. Auch bei bipolaren Anordnungen bietet der erfindungsgemäße Gegenstand die sichere Erfassung und Überwachung elektrochirurgischer Behandlungen.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 1: HF-Chirurgieeinrichtung
- 10: HF-Chirurgiegerät
- 11: HF-Generator
- 12: Eingangsanschluss
- 13: Erster Ausgangsanschluss
- 14: Zweiter Ausgangsanschluss
- 15: Steuerungseinrichtung
- 16: Recheneinrichtung
- 17: Speichereinrichtung
- 18: Signalverarbeitungseinrichtung
- 19: Anzeige
- 20: Spannungsmesseinrichtung
- 21: Strommesseinrichtung
- 22: Zeitmesseinrichtung
- 23: Temperaturmesseinrichtung
- 30: Elektrochirurgisches Instrument
- 31: Koagulationselektrode
- 32: Messelektrode
- 32': Messelektrode
- 33: Neutralelektrode
- 40: Messgewebebereich
- 41: Zielgewebebereich, Koagulationszone
- 42: Umliegendes Gewebe
- 50: Eingabeeinheit

- r: Radius des Messgewebebereichs/des Zielgewebebereichs, definierter Abstand
- U: Steuerungsleitung
- I: Steuerungsleitung
- T: Steuerungsleitung
- θ: Steuerungsleitung
- W: Steuerungsleitung
- C, C': Steuerungsleitung
- u: Spannungsabfall, Spannung
- i: Strom
- t: Zeitdauer
- ϑ: Temperatur
- w: Ermittelter Endwert
- c: Abschaltsignal
- R_{Z}: Widerstand Zielgewebebereich
- R_{M}: Widerstand Messgewebebereich
- R_{R}: Restwiderstand des Zielgewebebereichs
- R_{Ü}: Übergangswiderstand
- R_{P}: Widerstand Patient

## Patentansprüche

1. HF-Chirurgieeinrichtung zum Behandeln biologischen Gewebes mittels eines HF-Stromes, umfassend
- ein elektrochirurgisches Instrument (30) mit einer Koagulationselektrode (31) und
- ein HF-Chirurgiegerät (10) mit
-- einem HF-Generator (11) zum Erzeugen einer HF-Spannung und zum Zuführen des HF-Stromes zu der Koagulationselektrode (31) des elektrochirurgischen Instruments (30) und
-- mindestens einer Steuerungseinrichtung (15) zum Beenden des Koagulationsvorganges,
wobei der Steuerungseinrichtung (15) Folgendes zugeordnet ist:
- eine Messvorrichtung,
welche derart ausgebildet ist, dass sie mindestens einen Messgewebeenergieeintrag in einen definierten Messgewebebereich (40) beschreibenden Messwert (u, i, t, ϑ) erfasst,
wobei sie mindestens einen Messwert unmittelbar im Messgewebebereich mittels einer Messelektrode (32, 32') erfasst, wobei die Koagulationselektrode und die Messelektrode derart zueinander angeordnet sind, dass sie den Messgewebebereich hinsichtlich seiner Ausdehnung zumindest eindimensional in einer Richtung abgreifen,
- eine Recheneinrichtung (16), welche derart ausgebildet ist,
-- dass sie den Messgewebeenergieeintrag in den definierten Messgewebebereich (40) feststellt und
-- dass sie einen für den Koagulationsvorgang vorgegebenen Endwert (w) für einen Zielgewebeenergieeintrag in einen Zielgewebebereich (41) ermittelt und/oder als gespeicherten Endwert (w) übernimmt,
wobei die Steuerungseinrichtung (15) derart ausgebildet ist,
dass sie den HF-Generator (11) auf Basis des Endwertes (w) derart steuert oder regelt, dass der erforderliche HF-Strom dem Zielgewebebereich (41) zugeführt wird, und
dass sie dann, wenn der Zielgewebeenergieeintrag den Endwert (w) erreicht, ein Abschaltsignal (c) erzeugt.

2. HF- Chirurgieeinrichtung nach Anspruch 1,
**dadurch gekennzeichnet, dass**
die Recheneinrichtung (16) derart ausgebildet ist, dass der Endwert (w) für den Zielgewebeenergieeintrag in zeitlich definierten Abständen wiederholt neu ermittelt wird, so dass dieser entsprechend einem festen zeitlichen Ablauf einer Zeitplanregelung vorgegeben wird.

3. HF- Chirurgieeinrichtung nach Anspruch 1 oder 2, insbesondere nach Anspruch 1,
**dadurch gekennzeichnet, dass**
die Recheneinrichtung (16) derart ausgebildet ist, dass der Endwert (w) für den Zielgewebeenergieeintrag aus einem vorgegebenen Sollwertverlauf eingelesen wird, so dass dieser entsprechend einem festen zeitlichen Ablauf einer Zeitplanregelung vorgegeben wird.

4. HF- Chirurgieeinrichtung nach einem der vorhergehenden Ansprüche, **dadurch gekennzeichnet, dass**
der Messgewebebereich (40) kleiner ist als der Zielgewebebereich (41).

5. HF- Chirurgieeinrichtung nach einem der vorhergehenden Ansprüche, **dadurch gekennzeichnet, dass**
die Messvorrichtung
mindestens eine in einem definierten Abstand (r) zur Koagulationselektrode (31) angeordnete Messelektrode (32) umfasst, die mit dem zu behandelnden Gewebe im Messgewebebereich (40) in elektrisch leitenden Kontakt bringbar ist, sowie
mindestens eine Spannungsmesseinrichtung (20) zur Messung einer, unabhängig von etwaigen Leitungsverlusten oder dergleichen Verlusten, über dem Messgewebebereich (40) abfallenden Spannung (u),
so dass eine in den Messgewebebereich (40) eingebrachte Leistung aus den gemessenen Werten zur Ermittlung des Endwertes für den Zielgewebeenergieeintrag ermittelbar ist.

6. HF- Chirurgieeinrichtung nach einem der vorhergehenden Ansprüche, **dadurch gekennzeichnet, dass**
die Messvorrichtung ferner
mindestens eine Strommesseinrichtung (21) zur
Messung des HF-Stromes in dem Messgewebebereich (40) zur Ermittlung des Endwertes (w) für den Zielgewebeenergieeintrag aufweist.

7. HF- Chirurgieeinrichtung nach einem der vorhergehenden Ansprüche, **dadurch gekennzeichnet, dass**
die Messvorrichtung ferner
mindestens eine erste Zeitmesseinrichtung (22) zur Messung einer Dauer des Stromflusses in den Messgewebebereich (40) zur Ermittlung des Endwertes für den Zielgewebeenergieeintrag aufweist.

8. HF- Chirurgieeinrichtung nach einem der vorhergehenden Ansprüche, **dadurch gekennzeichnet, dass**
die Messvorrichtung
mindestens eine zweite Zeitmesseinrichtung zur Messung einer Dauer des Messgewebeenergieeihtrags in den Messgewebebereich (40) aufweist, so dass der Endwert (w) für den Zielgewebeenergieeintrag in Abhängigkeit von der Dauer (t) des Messgewebeenergieeintrags ermittelbar ist.

9. HF- Chirurgieeinrichtung nach einem der vorhergehenden Ansprüche, **dadurch gekennzeichnet, dass**
der Messvorrichtung eine Speichereinrichtung (17) zur Speicherung experimentell ermittelter Messreihen von Vergleichsprobengewebe beschreibenden Messwerten zugeordnet ist,
so dass für die Ermittlung des Endwertes (w) für den Zielgewebeenergieeintrag die gespeicherten Messreihen zugrunde gelegt werden.

10. HF- Chirurgieeinrichtung nach einem der vorhergehenden Ansprüche, insbesondere nach Anspruch 9,
**dadurch gekennzeichnet, dass**
die Messvorrichtung derart ausgebildet ist, dass für die Ermittlung des Endwertes (w) Messreihen von Wärmekapazitäten und/oder Wärmeleitfähigkeiten zugrunde gelegt werden.

11. HF- Chirurgieeinrichtung nach einem der vorhergehenden Ansprüche, insbesondere nach Anspruch 9 oder 10,
**dadurch gekennzeichnet, dass**
der Steuerungseinrichtung (15) eine weitere Messvorrichtung zugeordnet ist, welche derart ausgebildet ist, dass das zu behandelnde Gewebe beschreibende Messwerte zu deren Aufnahme als Vergleichswerte in die

12. HF- Chirurgieeinrichtung nach einem der vorhergehenden Ansprüche, insbesondere nach einem der Ansprüche 9 bis 11,
**dadurch gekennzeichnet, dass**
der Steuerungseinrichtung (15) eine Eingabeeinheit (50) zugeordnet ist, welche derart ausgebildet ist, dass ein Anwender die ein Vergleichsprobengewebe beschreibenden Messwerte und/oder bekannte Gewebeparameter zur Speicherung in die Speichereinrichtung (17) eingeben kann.

13. HF- Chirurgieeinrichtung nach einem der vorhergehenden Ansprüche, **dadurch gekennzeichnet, dass**
die Messvorrichtung ferner
mindestens eine Temperaturmesseinrichtung (23) zur Messung einer Gewebetemperatur (ϑ) in dem Messgewebebereich (40) aufweist,
so dass der Endwert (w) für den Zielgewebeenergieeintrag in Abhängigkeit von der gemessenen Temperatur (ϑ) oder in Abhängigkeit der gemessenen Temperatur (ϑ) und der gespeicherten Messreihen ermittelbar und/oder korrigierbar ist.

14. HF- Chirurgieeinrichtung nach einem der vorhergehenden Ansprüche, **dadurch gekennzeichnet, dass**
die Messvorrichtung eine Einrichtung zur Messung eines Übergangswiderstandes zwischen der Koagulationselektrode (31) und dem Messgewebebereich (40) zur Lieferung eines Korrekturwertes für die Ermittlung des Endwertes (w) für den Zielgewebeenergieeintrag aufweist.

15. HF- Chirurgieeinrichtung nach einem der vorhergehenden Ansprüche, insbesondere nach einem der Ansprüche 6 bis 14,
**dadurch gekennzeichnet, dass**
die Strommesseinrichtung (21) derart ausgebildet ist, dass der erfasste Strom (i) an die Steuerungseinrichtung (15) übermittelbar ist, so dass diese eine Phasenbeziehung zwischen der Spannung (u) und dem Strom (i) als Korrekturwert ermittelt.

16. HF- Chirurgieeinrichtung nach einem der vorhergehenden Ansprüche, insbesondere nach einem der Ansprüche 5 bis 15,
**dadurch gekennzeichnet, dass**
die Spannungsmesseinrichtung (20) als modularer Baustein der HF-Chirurgieeinrichtung (1) vorgesehen ist.

17. HF- Chirurgieeinrichtung nach einem der vorhergehenden Ansprüche, insbesondere nach einem der Ansprüche 6 bis 16,
**dadurch gekennzeichnet, dass**
die Strommesseinrichtung (21) als modularer Baustein der HF-Chirurgieeinrichtung (1) vorgesehen ist.

18. HF- Chirurgieeinrichtung nach einem der vorhergehenden Ansprüche, **dadurch gekennzeichnet, dass**
die Steuerungseinrichtung (15) derart ausgebildet ist, dass sie das Abschaltsignal (c) an den HF-Generator (11) übermittelt, so dass dieser abschaltet und so den HF-Strom abschaltet.

19. HF- Chirurgieeinrichtung nach einem der vorhergehenden Ansprüche, **dadurch gekennzeichnet, dass**
mindestens eine Signalverarbeitungseinrichtung (18) vorgesehen ist, der das Abschaltsignal (c) zuführbar ist, wobei die Signalverarbeitungseinrichtung (18) derart ausgebildet ist, dass mittels des Abschaltsignals (c) eine optische und/oder akustische Anzeige (19) derart ansteuerbar ist, dass das Abschalten des HF-Stromes aufgrund des Abschaltsignals (c) zur Benutzerführung anzeigbar ist.

20. HF- Chirurgieeinrichtung nach einem der vorhergehenden Ansprüche, **dadurch gekennzeichnet, dass**
die Koagulationselektrode (31) als Kugelelektrode ausgebildet ist.

21. HF- Chirurgieeinrichtung nach einem der vorhergehenden Ansprüche, **dadurch gekennzeichnet, dass**
die Messvorrichtung zwei von der Koagulationselektrode (31) mit einem definierten Abstand (r) angeordnete Messelektroden (32, 32') aufweist, die mit dem zu behandelnden Gewebe in elektrisch leitenden Kontakt bringbar sind,
wobei die Messelektroden (32, 32') entlang einer Linie mit der Koagulationselektrode (31) an dem elektrochirurgischen Instrument (30) angeordnet sind und wobei die Koagulationselektrode (31) zwischen den Messelektroden (32, 32') angeordnet ist.

22. HF- Chirurgieeinrichtung nach einem der vorhergehenden Ansprüche, insbesondere nach einem der Ansprüche 5 bis 21,
**dadurch gekennzeichnet, dass**
die Messelektrode (32, 32') an dem elektrochirurgischen Instrument (30) ortsveränderbar angeordnet ist.

## Claims

1. Electrosurgical apparatus for treating biological tissue by means of an RF current, comprising:
- an electrosurgical instrument (30) comprising a coagulation electrode (31) and
- an electrosurgical appliance (10) comprising
-- an RF generator (11) for generating an RF voltage and for supplying the RF current to the coagulation electrode (31) of the electrosurgical instrument (30) and
-- at least one control apparatus (15) for completing the coagulation process,
wherein the following is assigned to the control apparatus (15):
- a measuring device, which is embodied in such a way that it detects at least one measurement value (u, i, t, θ) describing a measured tissue energy influx in a defined measured tissue region (40),
wherein it detects at least one measurement value directly in the measured tissue region by means of a measuring electrode (32, 32'), wherein the coagulation electrode and the measuring electrode are arranged in such a way relative to one another that they tap the measured tissue region in respect of the extent thereof at least in a one-dimensional manner in one direction,
- a computer apparatus (16), which is embodied in such a way
-- that it determines the measured tissue energy influx in the defined measured tissue region (40) and
-- that it establishes a final value (w), which is predetermined for the coagulation process, for a target tissue energy influx in a target tissue region (41) and/or adopts said final value as a stored final value (w),
wherein the control apparatus (15) is embodied in such a way that it controls or regulates the RF generator (11) on the basis of the final value (w) in such a way that the required RF current is supplied to the target tissue region (41) and that it then generates a shutdown signal (c) when the target tissue energy influx reaches the final value (w).

2. Electrosurgical apparatus according to Claim 1,
**characterized in that**
the computer apparatus (16) is embodied in such a way that the final value (w) for the target tissue energy influx is repeatedly re-established at temporally defined intervals such that it is predetermined in accordance with fixed timing of a time scheduled closed-loop control.

3. Electrosurgical apparatus according to Claim 1 or 2, in particular according to Claim 1,
**characterized in that**
the computer apparatus (16) is embodied in such a way that the final value (w) for the target tissue energy influx is read from a predetermined setpoint value curve such that it is predetermined in accordance with fixed timing of a time scheduled closed-loop control.

4. Electrosurgical apparatus according to one of the preceding claims,
**characterized in that**
the measured tissue region (40) is smaller than the target tissue region (41).

5. Electrosurgical apparatus according to one of the preceding claims,
**characterized in that**
the measuring device
comprises at least one measuring electrode (32) arranged at a defined distance (r) from the coagulation electrode (31), which measuring electrode can be brought into electrically conductive contact with the tissue to be treated in the measured tissue region (40), and
at least one voltage measuring apparatus (20) for measuring a voltage drop (u) over the measured tissue region (40), independently of possible conduction losses or similar losses, such that a power introduced into the measured tissue region (40) is establishable from the measured values for establishing the final value for the target tissue energy influx.

6. Electrosurgical apparatus according to one of the preceding claims,
**characterized in that**
the measuring device furthermore has at least one current measuring apparatus (21) for measuring the RF current in the measured tissue region (40) for establishing the final value (w) for the target tissue energy influx.

7. Electrosurgical apparatus according to one of the preceding claims,
**characterized in that**
the measuring device furthermore has at least one first time measuring apparatus (22) for measuring a duration of the current flow in the measured tissue region (40) for establishing the final value for the target tissue energy influx.

8. Electrosurgical apparatus according to one of the preceding claims,
**characterized in that**
the measuring device has
at least one second time measuring apparatus for measuring a duration of the measured tissue energy influx into the measured tissue region (40) such that the final value (w) for the target tissue energy influx is establishable in a manner dependent on the duration (t) of the measured tissue energy influx.

9. Electrosurgical apparatus according to one of the preceding claims,
**characterized in that**
a storage apparatus (17) for storing experimentally established measurement series of measurement values describing comparison sample tissue is assigned to the measuring device such that the stored measurement series are used as a basis for establishing the final value (w) for the target tissue energy influx.

10. Electrosurgical apparatus according to one of the preceding claims, in particular according to Claim 9,
**characterized in that**
the measuring device is embodied in such a way that measurement series of thermal capacities and/or thermal conductivities are used as a basis for establishing the final value (w).

11. Electrosurgical apparatus according to one of the preceding claims, in particular according to Claim 9 or 10,
**characterized in that**
a further measuring device is assigned to the control apparatus (15), which measuring device is embodied in such a way that measurement values describing the tissue to be treated are measurable for the purposes of recording the latter as comparison values in the storage apparatus (17).

12. Electrosurgical apparatus according to one of the preceding claims, in particular according to one of Claims 9 to 11,
**characterized in that**
an input unit (50) is assigned to the control apparatus (15), which input unit is embodied in such a way that a user can enter the measurement values describing a comparison sample tissue and/or known tissue parameters for the purposes of storing these in the storage apparatus (17).

13. Electrosurgical apparatus according to one of the preceding claims,
**characterized in that**
the measuring device furthermore has at least one temperature measuring apparatus (23) for measuring a tissue temperature (θ) in the measured tissue region (40)
such that the final value (w) for the target tissue energy influx is establishable and/or correctable in a manner dependent on the measured temperature (θ) or in a manner dependent on the measured temperature (θ) and the stored measurement series.

14. Electrosurgical apparatus according to one of the preceding claims,
**characterized in that**
the measuring device has an apparatus for measuring a transfer resistance between the coagulation electrode (31) and the measured tissue region (40) for supplying a correction value for establishing the final value (w) for the target tissue energy influx.

15. Electrosurgical apparatus according to one of the preceding claims, in particular according to one of Claims 6 to 14,
**characterized in that**
the current measuring apparatus (21) is embodied in such a way that the detected current (i) is transmittable to the control apparatus (15) such that the latter establishes a phase relationship between the voltage (u) and the current (i) as a correction value.

16. Electrosurgical apparatus according to one of the preceding claims, in particular according to one of Claims 5 to 15,
**characterized in that**
the voltage measuring apparatus (20) is provided as a modular component of the electrosurgical apparatus (1).

17. Electrosurgical apparatus according to one of the preceding claims, in particular according to one of Claims 6 to 16,
**characterized in that**
the current measuring apparatus (21) is provided as a modular component of the electrosurgical apparatus (1).

18. Electrosurgical apparatus according to one of the preceding claims,
**characterized in that**
the control apparatus (15) is embodied in such a way that it transmits the shutdown signal (c) to the RF generator (11) such that the latter shuts down and thus switches off the RF current.

19. Electrosurgical apparatus according to one of the preceding claims,
**characterized in that**
provision is made for at least one signal processing apparatus (18), to which the shutdown signal (c) is suppliable, wherein the signal processing apparatus (18) is embodied in such a way that an optical and/or acoustic indication device (19) is actuatable by means of the shutdown signal (c) in such a way that the switching off of the RF current due to the shutdown signal (c) is indicatable for user guidance.

20. Electrosurgical apparatus according to one of the preceding claims,
**characterized in that**
the coagulation electrode (31) is embodied as a ball electrode.

21. Electrosurgical apparatus according to one of the preceding claims,
**characterized in that**
the measuring device has two measuring electrodes (32, 32') arranged at a defined distance (r) from the coagulation electrode (31), which measuring electrodes can be brought into electrically conductive contact with the tissue to be treated, wherein the measuring electrodes (32, 32') are arranged at the electrosurgical instrument (30) along a line with the coagulation electrode (31) and wherein the coagulation electrode (31) is arranged between the measuring electrodes (32, 32').

22. Electrosurgical apparatus according to one of the preceding claims, in particular according to one of Claims 5 to 21,
**characterized in that**
the measuring electrode (32, 32') is arranged at the electrosurgical instrument (30) with a modifiable position.

## Revendications

1. Dispositif chirurgical HF destiné à traiter un tissu biologique au moyen d'un courant HF, comprenant
- un instrument électrochirurgical (30) muni d'une électrode de coagulation (31) et
- un appareil chirurgical HF (10) muni
-- d'un générateur HF (11) destiné à générer une tension HF et à acheminer le courant HF à l'électrode de coagulation (31) de l'instrument électrochirurgical (30) et
-- au moins un dispositif de commande (15) destiné à mettre fin au processus de coagulation, ce qui suit étant associé au dispositif de commande (15) :
- un système de mesure qui est configuré de telle sorte qu'il collecte au moins une valeur mesurée (u, i, t, ϑ) décrivant un apport d'énergie de tissu de mesure dans une zone de tissu mesuré (40),
celui-ci collectant au moins une valeur mesurée directement dans la zone de tissu mesuré au moyen d'une électrode de mesure (32, 32'),
l'électrode de coagulation et l'électrode de mesure étant disposées de telle sorte l'une par rapport à l'autre qu'elles mesurent la zone de tissu mesuré du point de vue de son expansion au moins de manière unidimensionnelle dans une direction,
- un dispositif de calcul (16) qui est configuré de telle sorte
-- qu'il établit l'apport d'énergie de tissu de mesure dans la zone de tissu mesuré (40) et
-- qu'il détermine une valeur finale (w) prédéfinie pour le processus de coagulation pour un apport d'énergie de tissu cible dans une zone de tissu cible (41) et/ou la réceptionne en tant que valeur finale (w) mise en mémoire,
le dispositif de commande (15) étant configuré de telle sorte
qu'il commande ou régule le générateur HF (11) sur la base de la valeur finale (w) de telle sorte que le courant HF nécessaire est acheminé à la zone de tissu cible (41) et
qu'il génère un signal de mise hors circuit (c) lorsque l'apport d'énergie de tissu cible atteint la valeur finale (w).

2. Dispositif chirurgical HF selon la revendication 1, **caractérisé en ce que** le dispositif de calcul (16) est configuré de telle sorte que la valeur finale (w) pour l'apport d'énergie de tissu cible est de nouveau déterminée de manière répétitive à des intervalles de temps définis, de sorte que celle-ci est prédéfinie conformément à un déroulement dans le temps fixe d'une régulation à programme.

3. Dispositif chirurgical HF selon la revendication 1 ou 2, en particulier selon la revendication 1, **caractérisé en ce que** le dispositif de calcul (16) est configuré de telle sorte que la valeur finale (w) pour l'apport d'énergie de tissu cible est relevée à partir d'une courbe de valeur de consigne prédéfinie, de sorte que celle-ci est prédéfinie conformément à un déroulement dans le temps fixe d'une régulation à programme.

4. Dispositif chirurgical HF selon l'une des revendications précédentes, **caractérisé en ce que** la zone de tissu mesuré (40) est plus petite que la zone de tissu cible (41).

5. Dispositif chirurgical HF selon l'une des revendications précédentes, **caractérisé en ce que** le système de mesure
comprend au moins une électrode de mesure (32) disposée à un écart (r) défini par rapport à l'électrode de coagulation (31), laquelle peut être amenée en contact électriquement conducteur avec le tissu à traiter dans la zone de tissu mesuré (40), ainsi
qu'au moins un dispositif de mesure de tension (20) destiné à mesurer une tension (u) qui chute au niveau de la zone de tissu mesuré (40) indépendamment des éventuelles pertes en ligne ou pertes similaires,
de sorte qu'une puissance introduite dans la zone de tissu mesuré (40) peut être déterminée à partir des valeurs mesurées en vue de déterminer la valeur finale pour l'apport d'énergie de tissu cible.

6. Dispositif chirurgical HF selon l'une des revendications précédentes, **caractérisé en ce que** le système de mesure possède en outre au moins un dispositif de mesure de courant (21) destiné à mesurer le courant HF dans la zone de tissu mesuré (40) en vue de déterminer la valeur finale (w) pour l'apport d'énergie de tissu cible.

7. Dispositif chirurgical HF selon l'une des revendications précédentes, **caractérisé en ce que** le système de mesure possède en outre au moins un premier dispositif de mesure de temps (22) destiné à mesurer une durée du flux de courant dans la zone de tissu mesuré (40) en vue de déterminer la valeur finale pour l'apport d'énergie de tissu cible.

8. Dispositif chirurgical HF selon l'une des revendications précédentes, **caractérisé en ce que** le système de mesure possède au moins un deuxième dispositif de mesure de temps destiné à mesurer une durée de l'apport d'énergie de tissu de mesure dans la zone de tissu mesuré (40), de sorte que la valeur finale (w) pour l'apport d'énergie de tissu cible peut être déterminée en fonction de la durée (t) de l'apport d'énergie de tissu de mesure.

9. Dispositif chirurgical HF selon l'une des revendications précédentes, **caractérisé en ce qu'**au système de mesure est associé un dispositif à mémoire (17) destiné à mettre en mémoire des valeurs mesurées décrivant des séries de mesures déterminées de manière expérimentale d'échantillons de tissu comparatifs, de sorte que les séries de mesures mises en mémoire peuvent servir de base pour la détermination de la valeur finale (w) pour l'apport d'énergie de tissu cible.

10. Dispositif chirurgical HF selon l'une des revendications précédentes, notamment selon la revendication 9, **caractérisé en ce que** le système de mesure est configuré de telle sorte que la détermination de la valeur finale (w) est effectuée en se basant sur des séries de mesure de capacités thermiques et/ou de conductivités thermiques.

11. Dispositif chirurgical HF selon l'une des revendications précédentes, notamment selon la revendication 9 ou 10, **caractérisé en ce qu'**un système de mesure supplémentaire est associé au dispositif de commande (15), lequel est configuré de telle sorte que les valeurs mesurées décrivant le tissu à traiter peuvent être mesurées en vue d'être enregistrées en tant que valeurs comparatives dans le dispositif à mémoire (17).

12. Dispositif chirurgical HF selon l'une des revendications précédentes, notamment selon l'une des revendications 9 à 11, **caractérisé en ce qu'**une unité de saisie (50) est associée au dispositif de commande (15), laquelle est configurée de telle sorte qu'un utilisateur peut saisir les valeurs mesurées décrivant un échantillon de tissu comparatif et/ou des paramètres de tissu connus en vue de leur mise en mémoire dans le dispositif à mémoire (17).

13. Dispositif chirurgical HF selon l'une des revendications précédentes, **caractérisé en ce que** le système de mesure possède en outre au moins un dispositif de mesure de température (23) destiné à mesurer une température de tissu (ϑ) dans la zone de tissu mesuré (40), de sorte que la valeur finale (w) pour l'apport d'énergie de tissu cible peut être déterminée et/ou corrigée en fonction de la température mesurée (ϑ) ou en fonction de la température mesurée (ϑ) et des séries de mesures mises en mémoire.

14. Dispositif chirurgical HF selon l'une des revendications précédentes, **caractérisé en ce que** le système de mesure possède un dispositif servant à mesurer une résistance de contact entre l'électrode de coagulation (31) et la zone de tissu mesuré (40) en vue de délivrer une valeur de correction pour la détermination de la valeur finale (w) pour l'apport d'énergie de tissu cible.

15. Dispositif chirurgical HF selon l'une des revendications précédentes, notamment l'une des revendications 6 à 14, **caractérisé en ce que** le dispositif de mesure de courant (21) est configuré de telle sorte que le courant (i) détecté peut être transmis au dispositif de commande (15), de sorte que celui-ci détermine une relation de phase entre la tension (u) et le courant (i) en tant que valeur de correction.

16. Dispositif chirurgical HF selon l'une des revendications précédentes, notamment l'une des revendications 5 à 15, **caractérisé en ce que** le dispositif de mesure de tension (20) est présent sous la forme d'un composant modulaire du dispositif chirurgical HF (1).

17. Dispositif chirurgical HF selon l'une des revendications précédentes, notamment l'une des revendications 6 à 16, **caractérisé en ce que** le dispositif de mesure de courant (21) est présent sous la forme d'un composant modulaire du dispositif chirurgical HF (1).

18. Dispositif chirurgical HF selon l'une des revendications précédentes, **caractérisé en ce que** le dispositif de commande (15) est configuré de telle sorte qu'il transmet le signal de mise hors circuit (c) au générateur HF (11), de sorte que celui-ci se met hors circuit et coupe ainsi le courant HF.

19. Dispositif chirurgical HF selon l'une des revendications précédentes, **caractérisé en ce qu'**il existe au moins un dispositif de traitement de signal (18) auquel peut être acheminé le signal de mise hors circuit (c), le dispositif de traitement de signal (18) étant configuré de telle sorte que le signal de mise hors circuit (c) permette de commander un indicateur (19) visuel et/ou sonore de telle sorte que la coupure du courant HF du fait du signal de mise hors circuit (c) peut être indiquée en tant que guide d'utilisateur.

20. Dispositif chirurgical HF selon l'une des revendications précédentes, **caractérisé en ce que** l'électrode de coagulation (31) est réalisée sous la forme d'une électrode à boule.

21. Dispositif chirurgical HF selon l'une des revendications précédentes, **caractérisé en ce que** le système de mesure possède deux électrodes de mesure (32, 32') disposées à un écart (r) défini par rapport à l'électrode de coagulation (31), lesquelles peuvent être amenées en contact électriquement conducteur avec le tissu à traiter,
les électrodes de mesure (32, 32') étant disposées le long d'une ligne avec l'électrode de coagulation (31) sur l'instrument électrochirurgical (30) et l'électrode de coagulation (31) étant disposée entre les électrodes de mesure (32, 32').

22. Dispositif chirurgical HF selon l'une des revendications 5 à 21, **caractérisé en ce que** l'électrode de mesure (32, 32') est disposée sur l'instrument électrochirurgical (30) de manière mobile.
